# EUROPEAN PATENT APPLICATION

(11) **EP 4 667 555 A1**
(43) Date of publication of application: **24.12.2025**
(21) Application number: 24182686.6
(22) Date of filing: 17.06.2024
(51) Int. Cl.: C12M 1/00

(54) **ELEMENTS, SYSTEMS, AND METHODS FOR ILLUMINATION**

(71) Applicant: Gehring, Timo, 66424 Homburg (DE); Maurer, Patrick, 66540 Neunkirchen (DE)
(72) Inventor: Gehring, Timo, 66424 Homburg (DE); Maurer, Patrick, 66540 Neunkirchen (DE)
(74) Representative: Stellbrink & Partner Patentanwälte mbB

(57) **Abstract**

The present invention relates to use of an illumination element (10) in a photobioreactor. The illumination element is adapted for illuminating an interior of a container (40) The illumination element comprises a light source, and a battery configured to supply energy to the light source, wherein the battery is a rechargeable battery. The present invention also relates to a supply station for supplying energy to the illumination element, to a use of a system in a photobioreactor, wherein the system is adapted for illuminating an interior of a container, wherein the system comprises a plurality of the illumination elements, to a method for illuminating an interior of a container comprising providing a plurality of the illumination elements.

## Description

The present invention relates generally to the field of biological cultivation. More particularly, it relates to illumination elements for facilitating biological growth, and to systems and methods for using the illumination elements in a container.

The cultivation of algae may enable sustainable production of biomass and valuable materials that were previously obtained from fossil sources. Currently, 10% of the world's oil production is used for the production of plastics and as raw material for the chemical industry. Algae can make a significant contribution to establishing a carbon-based circular economy for humanity that may be, at least significantly, independent of crude oil if they can be cultivated on a large industrial scale all year round. In particular, cultivation independent of location with high yield and with green energy may be of advantage. Overexploitation of nature (e.g., algae harvesting in the sea) may also not be a sustainable option for the preservation of diversified ecosystems.

Embodiments of the present invention relate to a new type of photobioreactor for the cultivation of algae with internal illumination. The present technology may have particular advantages compared to currently available design variants of photobioreactors and may be easily scalable. As described herein, embodiments of the present technology may involve realization of internal lighting in a vessel, container, tank, or reactor for algae cultivation that may make it possible to easily transform vessels, tanks, and containers that may have been previously used for other purposes into photobioreactors. Thus, embodiments of the present technology can be particularly advantageous for algae cultivation.

Typical photobioreactors may be divided into the following categories:
1) Outdoor photobioreactors that operate with sunlight. These photobioreactors may exist as outdoor systems with the sun as the main light source, possibly with additional artificial lighting. These outdoor photobioreactors may have the disadvantage that their operating time may be limited to the duration of sunshine. In particular, they may not allow round-the-clock production/cultivation of algae. In other words, such photobioreactor systems for algae cultivation are dependent on the sun. When illuminated by the sun, the maximum production time may, thus, be limited to the duration of sunshine and may not be fully continuous all year round (8760 hours per year).
2) Indoor photobioreactors that can be illuminated from outside or inside with artificial lighting. Indoor photobioreactors may work without sunlight and only with artificial lighting, thus enabling fully continuous production 24 hours a day, 365 days a year. Thus, large-scale closed systems with internal lighting for algae cultivation may be realized with internal or external lighting.

Indoor photobioreactors with external lighting may comprise, for example, tubular reactors, pool reactors, plate reactors, and zig-zag reactors, where the lighting is installed externally into the reactor. In artificially illuminated photobioreactors with external lighting, the layer thickness of the systems, may however, be limited to 5-15 cm, making the scaling of such systems to large volumes technically complex, expensive, and inflexible.

The penetration depth of the light, generally, depends on the growth of the algae. At first, when the volume of algae in the system is low, there may be an excess of light. Once the algae culture has grown strongly, there may be too little light for continuous algae growth. In densely grown algae cultures, the culture solution may become optically dense after 5-15 cm, i.e. no more light may arrive after this distance.

Therefore, indoor photobioreactors may be significantly limited in size and design variety of the large-scale systems, especially with regard to the simplest possible scale-up. For example, with external lighting, the systems cannot be thick (in a direction perpendicular to the light source), as the penetration depth of light in a dense algae culture may typically be 5-15 cm. The scaling and cultivation of large volumes may, thus, be technically complex and/or require a lot of space.

Photobioreactors such as tubular reactors may also have other disadvantages, e.g., gas exchange due to gradient formation in the long tubes (O₂, CO₂).

Indoor photobioreactors with internal lighting that operate with permanently installed tubes (horizontal or vertical) immersed into a reaction medium of a reactor may typically contain built-in tubes that contain lighting elements. These designs may also not be easily scalable, as a large number of illumination tubes may be required with increasing container size and these must withstand the static and dynamic forces of large container dimensions or volumes. Further, the cleaning of photobioreactors with built-in parts, in particular internal fixtures such as permanently installed internal lighting units, may be complex and technically demanding.

Overall, known photobioreactor types may be technically complex and the maximum reactor size may be limited, making the production of algae complicated, in particular at large scale. A particular disadvantage of known photobioreactors using permanently installed lighting units may be the lack of scalability, especially for large cultivation volumes such as exceeding 10, 100, or 1000 m³, as a large number of lighting tubes may be required as the container size increases and these may have to withstand the static and dynamic forces of large container dimensions or volumes.

Glemser et. al., Application of light-emitting diodes (LEDs) in cultivation of phototrophic microalgae: current state and perspectives, Appl. Microbiology and Biotechnology, Vol. 100, pages 1077 - 1088 (2016), provides an overview on the application of LEDs in microalgal cultivation processes. The authors particularly focus on the use of narrow-wavelength LEDs to address fundamental as well as applied aspects of light color on algae biomass and value-added compound formation. The application of internal and external illumination systems is further reviewed together with trends in the industrial use of LED systems to intensify algae process efficiency.

Heining et. al., Internal illumination of photobioreactors via wireless light emitters: a proof of concept, Journal of Applied Phycology, Vol. 27, pages 59 - 66 (2015), discloses an illumination system based on free, suspend-able, light emitters, which are powered wirelessly by near-field resonant inductive coupling and, therefore, are able to illuminate a photobioreactor more homogeneously than external illumination systems. The authors conclude that the internal illumination via wireless light emitters results in a more uniform light distribution and a higher average light intensity in the reactor.

Heining et. al., Photobioreactors with internal illumination - A survey and comparison, Biotechnology Journal (2015), reviews different internally illuminated photobioreactors and compares them based on the ratio of illuminated surface-to-culture volume and the occupied volume by internal light-emitting elements.

US2014113362A1 discloses a closed algae cultivation system that has an algae supplier, a first conduit, at least two containers, and at least two light light-emitting devices. The algae supplier is coupled to the at least two containers via the first conduit. Each container includes at least one sidewall, a bottom and an upper lid, which together define an accommodating volume for accommodating an algal stock culture from the algae supplier. Each container is made of at least one opaque material and includes a first opening disposed on one of the at least one sidewall, and a second opening disposed at the bottom. The at least two light-emitting devices are respectively coupled to the upper lid of each container and extend therefrom along the longitudinal direction to the container such that at least a portion of the light light-emitting devices is submerged in the liquid algal stock culture.

EP2719753B1 discloses a system for illuminating the interior of a photobioreactor by means of small spheres (<= 1 cm) that may be charged from the outside by an electromagnetic field. However, in this system, the illumination may not be realized continuously, but only by light flashes, since the energy supply for electric fields cannot be realized continuously, but may only be discontinuous based on a frequency of the external electric field. Further, the external electromagnetic field may have a negative effect on algae growth. Moreover, an electromagnetic field may not be applied in a metallic stainless-steel container or on an industrial scale. Additionally, any metallic or electrical installations such as sensors and/or safety components may not be installed inside the spheres or in the reaction chamber due to interference from the external electromagnetic field. Further, due to the exponentially decreasing field strength away from the source of the electromagnetic field, spheres closer to the source may receive significantly more energy/electric field than spheres farther away. The system as disclosed in EP2719753B1 may, therefore, be difficult to scale to large containers and to metallic containers.

The present invention seeks to overcome or at least alleviate the shortcomings and disadvantages of the prior art. It is an object of the present technology to allow the realization of a photobioreactor with internal illumination. More particularly, it is an object of the present invention to allow the lighting unit to be physically separated from the container. The physical separation of the lighting unit may make it technically easier and simpler to scale up algae cultivation. In addition, empty containers may easily be transformed into photobioreactors.

According to a first aspect, the present invention relates to an illumination element for illuminating an interior of a container, the illumination element comprising: a light source, and a battery configured to supply energy to the light source.

The illumination element may be used for different types of container: container, reactor, tank, basin, natural basin, storage container, storage tank, artificial basin, swimming pool, lake, river, sea, cavern, or a cave.

The illumination element may, thus, not need to be supplied with energy externally during operation. Instead, the battery may power the light source, and may be recharged once it runs out. Thus, embodiments of the present technology may be of particular advantage if continuous energy supply is not available (for example, in remote locations or if energy is derived from renewable sources).

The illumination element may comprise a plurality of light sources.

A wavelength of light emitted by a first of the plurality of light sources may be different from a wavelength of light emitted by a second of the plurality of light sources. In other words, the plurality of light sources may be configured to emit light of at least two wavelengths. The ability to emit light at multiple wavelengths may be of advantage in promoting/suppressing growth of algae. For example, light at one wavelength that promotes growth may be used in an initial stage of cultivation, whereas light at another wavelength may be used to boost growth at a later stage of cultivation or to induce product formation or to suppress growth.

In particular, the different wavelengths may allow production of a value-added product with algae in a two-stage process. For example, first, algae may be allowed to grow as fast as possible to generate high amounts of biomass. Then cultivation conditions may be changed to force the algae to generate as much of the value-added product inside as possible. The algae may then be harvested or their cultivation stopped to extract the value-added product.

A wavelength of light emitted by any one of the plurality of light sources may, at least significantly, be identical to a wavelength of light emitted by any other of the plurality of light sources.

The illumination element may be configured to be connected to a supply station.

It will be understood that a connection may in particular refer to an electrical connection to transfer energy.

The illumination element may be configured to be attracted and/or repelled by a supply station, and wherein the illumination element may comprise an attachment element configured to allow the illumination element to be attracted and/or repelled by the supply station. In other words, the illumination element may be configured such that the supply station may exert a force on the illumination element. The driving of the illumination element by the supply station may be of advantage in positioning the illumination element within a reaction medium of the container as well as in facilitating replacement of illumination elements with low remaining battery capacity, as described later. Thus, the attachment element may be of particular advantage in optimum and robust illumination of the container.

The attachment element may comprise a magnetic material. In particular, the attachment element may comprise a magnet. As described later, the magnet may, particularly advantageously, comprise an electromagnet, that may allow the positioning of the illumination element within a reaction medium of the container.

The battery may be a rechargeable battery.

The battery may be configured to receive energy from the supply station.

The battery may comprise any of a Li-ion battery, a Li-polymer battery, a solid-state battery, a super capacitor battery, an Ni-Cd battery, an Ni-Metal Hydride battery, a redox flow battery, a sodium ion battery, and a lithium-sulphur solid-state battery.

The energy may be received wirelessly.

The energy may be received inductively.

The illumination element may comprise an illumination element induction coil.

The battery may be configured to receive energy from the supply station in a configuration such that a minimum distance between the illumination element, or at least a part thereof, and the supply station, or at least a part thereof, may be less than 1 m, preferably less than 10 cm, further preferably less than 1 cm. In particular, the energy transfer between the supply station and the illumination element may take place when the illumination element is in contact with the supply station.

A rate at which energy may be received inductively may be at least 10 W, preferably at least 50 W, further preferably at least 100 W. Owing to the smaller distance between the illumination element and the supply station as described above, a higher rate of energy transfer may be realized. This may be of particular advantage in improving an efficiency of the system, as illumination elements may be quickly recharged and be made available for release into a reaction medium of the container. The increased efficiency may be of particular advantage in later stages of cultivation when a relatively (compared to initial stages of cultivation) large number of illumination elements may be needed.

The illumination element may comprise a rectifier configured to convert an alternating current to a direct current.

The rectifier may be connected to the illumination element induction coil.

The rectifier may be connected to the battery such that the direct current can be used to charge the battery. In other words, the terminals of the battery may be appropriately connected to those of the rectifier to allow charging of the battery.

The energy may be received over a wired connection.

The illumination element may comprise a plug complementary to a socket of the supply station. A plug may allow, for example, even higher rates of energy transfer than achieved with close-contact wireless transfer as described above. However, including a plug may be more complex due to a waterproofing requirement of the plug and other electrical connections.

The rectifier, as described above, may be connected between the plug and the battery, thus allowing the illumination element to be charged by means of an alternating current as provided, for example, by a local grid.

The illumination element may comprise a housing.

The housing may comprise any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material. The housing may comprise material that may be rigid or flexible (inflatable/expandable/stretchable). In particular, the housing may comprise a material that may be, at least partially, inert and/or impermeable to a reaction medium of the container.

The housing may comprise a plurality of housing sections, such as two housing sections. However, in some embodiments, more than two housing sections may also be provided. A plurality of housing sections may be of advantage in allowing access to internal components of the illumination element for maintenance, etc. Two housing sections may allow for a simpler construction as only one joint between the two housing sections may be used to satisfy waterproofing and/or pressure constraints that may arise from immersion of the illumination element in a reaction medium of the container.

The two or more housing sections may be configured to be releasably attached to each other.

The two housing sections may be configured to be attached to each other by means of a thread connection.

The two housing sections may be configured to be attached to each other by means of a bayonet connection.

The housing may comprise, at least significantly, any of a spherical, an oval, a cylindrical, an angular, a polygonal, or a platonic solid shape.

Each of the two housing sections may comprise, at least significantly, a hemispherical shape.

The housing may, at least partially, be transparent to the light emitted by the light source.

The container may comprise a reaction medium, wherein the illumination element may be configured to be, at least partially, submerged in the reaction medium.

The housing may, at least partially, be impermeable to the reaction medium.

The light source may be embedded in the housing.

The light source may be arranged within the housing.

The light source may be arranged on an exterior of the housing. For example, the light source may comprise a bead of LEDs that may be wrapped around the housing.

The container may comprise a reaction medium, wherein the light source arranged on the exterior of the housing may, at least partially, be inert and/or impenetrable to the reaction medium. In particular, the light source may be impenetrable to the reaction medium under pressure expected at depths at which the illumination element may be deployed. For example, a maximum expected pressure may be considered to determine suitability of the light source.

The illumination element may comprise a data processing unit.

The illumination element may comprise a control unit, wherein the control unit may be configured to control the light source.

Controlling the light source may be understood to comprise controlling a state of the light source, i.e., switching the light source on or off. Controlling may also be understood to comprise controlling a wavelength and/or an intensity and/or a flashing frequency of the light source.

The control unit may be configured to control each of the plurality of light sources.

The control unit may be configured to receive data from the data processing unit.

The control unit may be configured to control the light source based, at least in part, on the data received from the data processing unit.

The illumination element may comprise a communication unit configured to send data to and/or receive data from an external device/element. Communication with an external element may comprise, in particular, communication with another illumination element. Communication with another illumination element may be of advantage, for example, in allowing the illumination elements to detect each other and exchange data and decide, e. g., autonomously to change position or lighting behavior.

The communication unit may be configured to send the data wirelessly. For example, data may be sent by means of acoustic and/or electromagnetic radiation, wherein a suitable wavelength may be chosen for the electromagnetic radiation.

The communication unit may be configured to receive data from the data processing unit.

The communication unit may be configured to send data to the data processing unit.

The illumination element may comprise a sensor.

The sensor may be configured to send data to the data processing unit.

The sensor may comprise a position and/or an orientation sensor. The position sensor may be configured to determine a position of the illumination element relative to the container and/or relative to another illumination element. Further, the position sensor may be configured to determine an absolute position of the illumination element. For example, the position sensor may comprise any of a GPS sensor, a sensor based on acoustic radiation (such as a sonar) and/or on electromagnetic radiation, with an appropriate wavelength (such as a wavelength range with high permeability in aqueous media, such as visible light (400-800 nm), near infrared range (800 - 2500 nm) or radio waves (> 1mm)) to allow for transmission through a reaction medium of the container. The orientation sensor may be of advantage, for example, in determining an orientation of an axis of the illumination element, such as a north-south axis for a spherical housing of the illumination element.

The sensor may comprise a temperature sensor. The temperature sensor may be configured to measure a temperature within the illumination element. The temperature sensor may be configured to measure a temperature of an environment of the illumination element. In particular, for example, two temperature sensors may be installed - one for measuring a temperature inside the illumination element and one for measuring a temperature outside. The temperature sensors may be of particular advantage in regulating a temperature of the illumination element, for example. As described later, the illumination element may also comprise a heat exchanger that may be configured to regulate a temperature inside the illumination element. The temperature sensor(s) may then be used to provide data based, at least in part, on which, the heat exchanger may regulate the temperature.

The sensor may comprise a pH sensor.

The sensor may comprise a pressure sensor.

The sensor may comprise a CO₂ concentration sensor.

The sensor may comprise a brightness sensor. The brightness sensor may be of particular advantage in monitoring a growth of algae in an environment of the illuminated element. For example, dense growth of algae in the environment may lead to a lower sensed brightness. The illumination element, particularly the data processing unit thereof, may be configured, for example, to switch a wavelength of light emitted by the light source to suppress growth if the sensed brightness falls below a defined threshold.

The sensor may comprise at least one of a(n): O₂ concentration sensor, viscosity sensor, turbidity sensor, density sensor, conductivity sensor, impedance sensor, optical density sensor, photosynthetically active radiation (PAR) sensor, camera, microscope, IR/Raman-spectroscopy-sensor, cell potential sensor, potentiostat sensor, cell cytometry sensor, specific ion-sensor (e. g., Na⁺, Cl⁻), specific nutrient concentrations sensor, photometer sensor, proximity sensor (to detect a wall of the container), and proximity sensor / distance sensor between two illumination elements. A proximity sensor may comprise, for example, a time-of-flight sensor.

The sensor may comprise a battery capacity sensor configured to measure a remaining capacity of the battery.

The communication unit may be configured to send data related, at least in part, to the remaining capacity of the battery.

The data processing unit may comprise a sensor threshold associated with the sensor.

The data processing unit may be configured, in response to the data received from the sensor crossing the sensor threshold, to send to the communication unit data related, at least in part, thereto. The data processing unit may be further configured to perform a defined action in response to the data crossing the sensor threshold. For example, as described above, the data processing unit may switch the wavelength of light emitted by the light source in response to the brightness or cell density falling below a threshold.

The sensor may be configured to detect a signal from an environment of the illumination element, and wherein the housing is, at least partially, transparent to the signal. The signal may, in particular, comprise a radiative signal, i.e., a signal that is transmitted/received via radiation, such as electromagnetic radiation.

A density of the illumination element may, at least significantly, be similar to a density of the reaction medium, or at least a part thereof. The similarity of density may allow the illumination element to be moved easily within the reaction medium.

The illumination element may comprise a density control unit configured to control a density of the illumination element.

The density control unit may be configured to receive data from the data processing unit.

The density control unit may be configured to control the density based, at least in part, on the data received from the data processing unit.

The density control unit may be configured to control the density based, at least in part, on the data received from the sensor. For example, the data received may relate, at least in part, to an optical density of the reaction medium in the immediate vicinity of the illumination element. Generally, as algae grows in the reaction medium, the density (and also the optical density) is of the reaction medium is expected to increase. By measuring the optical density, the illumination element may, thus, estimate the density of the reaction medium.

The density control unit may be configured to control the density to within 25%, preferably within 20%, further preferably within 15%, of the density of the reaction medium. Typical densities of the reaction medium may vary with respect to fresh water within the ranges defined above. For example, water has a density of 1.00 g/L at 20°C. Typical sea water of the oceans has a density of 1.02 to 1.03 g/L at 20°C depending on salt concentration (PSU), with higher densities up to 1.3 g/L (e.g., 33% m/m NaCl-concentration in the dead sea). Experts know that the density also depends on the temperature.

Moreover, due to possible gas injection into the photobioreactor, buoyancy can deviate from the buoyancy in pure liquid medium due to gas bubbles above/below the illumination elements and/or liquid currents due to pumping. Algae growth may also change the density of the culture medium and the buoyancy. Biofilm formation on the illumination elements may also change buoyancy of the illumination elements.

The density control unit may comprise a pump.

The density control unit may comprise a membrane.

The membrane may comprise a flexible material.

The density control unit may comprise a movable cylinder.

The density control unit may comprise a storage tank configured to store a fluid.

The density control unit may be configured to draw liquid into the storage tank.

The density control unit may be configured to pump liquid out of the storage tank.

The container may comprise a reaction medium, and wherein the illumination element may be configured to introduce an additive into the reaction medium. For example, the illumination element may comprise an additive dispensing unit. The additive dispensing unit may comprise, for example, a reservoir and a pump coupled to the reservoir.

The density of the illumination element may be changed by the dispensation of the additive. The density control unit, as described above, may be further configured, for example, to maintain a density of the illumination element after the additive has been dispensed.

The additive may comprise at least one of a gas, a nutrient, or a chemical.

The additive may comprise any of CO₂, O₃, and H₂O₂. Ozone (O₃) and hydrogen peroxide (H₂O₂) may be particularly advantageous for sanitization or oxidative stress induction purposes.

The illumination element may comprise a sampling unit configured to facilitate sample analysis. Analysis of samples may be of advantage in monitoring a progress of algae growth in the container.

The container may comprise a reaction medium, and wherein the illumination element, particularly the sampling unit thereof, may be configured to draw and store a sample from the reaction medium. In particular, the illumination element, particularly the sampling unit thereof, may be configured to draw and store at least one or a plurality of samples from the reaction medium.

The illumination element, particularly the sampling unit thereof, may be configured to release a stored sample into a supply station.

The battery may comprise a storage capacity in the range defined by 0.005 and 50 kWh per m² of illuminated surface area of the illumination element, preferably by 0.1 and 25 kWh per m², further preferably by 1 and 10 kWh per m².

A wavelength of at least one of the plurality of light sources may be in the range defined by 150 and 1000 nm, preferably by 400 and 800 nm.

A wavelength of at least one of the plurality of light sources may be in the range defined by 150 and 450 nm, preferably by 180 and 400 nm, further preferably by 200 and 380 nm.

Alternatively, or additionally, a wavelength of at least one of the plurality of light sources may be in the range defined by 350 and 900 nm, preferably by 370 and 850 nm, further preferably by 380 and 800 nm.

Alternatively, or additionally, a wavelength of at least one of the plurality of light sources may be in the range defined by 700 and 1200 nm, preferably by 750 and 1100 nm, further preferably by 800 and 1000 nm.

Different wavelengths may provide different advantages for algae cultivation. For example, wavelengths in the range 380-800 nm may correspond to typical photosynthetically active radiation, those in the range 200-380 nm (UV light) may be of advantage for light stress induction or sanitization, whereas those in the range 800-1000 nm (Infrared light) may be of advantage for growth boosting or for stress.

In other words, different wavelengths may have different purposes, such as normal growth, switching on/off genes, inducing reactions, or simulating stress environment.

The light source may be configured to emit light with a light intensity generated on the surface of the illumination element in the range defined by 1 and 5,000 µmol/m²/s, preferably by 2 and 4,000 µmol/m²/s, further preferably by 5 and 3,000 µmol/m²/s.

The light source may comprise a light emitting diode (LED).

The LED may comprise an organic LED (OLED).

The light source may comprise a quantum dot.

An efficiency of the LED may be at least 10%, preferably at least 30%, further preferably at least 50%. The efficiency may be a function, however, of the wavelength and/or the temperature. It may be understood that the efficiencies described above may be the efficiency of the LED at least for the wavelengths and the temperatures described herein, or as known to the skilled person.

The light efficacy of modern LEDs may also be denoted in units of µmol photons per Joule (µmol/J) and for LEDs suitable for the illumination element may be in the range of 2.0 - 9.0 µmol/J (the theoretical limit in case of 100% efficacy is 1.67 µmol/J for 200 nm wavelength and 8.4 µmol/J for 1000 nm wavelength).

The light source may comprise a laser such as an organic solid-state laser.

The container may be used for cultivation of algae, wherein a volume of the illumination element may be based, at least in part, on a density and/or type of the algae.

A volume of illumination element may be based, at least in part, on a number density of illumination elements to be used in the container.

A volume of the illumination element may be in the range defined by 100 and 600,000 cm³, preferably by 250 and 30,000 cm³, further preferably by 500 and 15,000 cm³. In other words, illumination elements that are spherical in shape may have a diameter of 10 cm, 30 cm, or 1 m, for example.

The illumination element may be configured to fluoresce.

The illumination element may comprise a fluorescent tag, the fluorescent tag comprising fluorescent paint.

The housing may comprise an external surface, the external surface in contact with a reaction medium of the container, and an internal surface opposite the external surface.

The fluorescent tag may be attached to the external surface.

The control unit may be configured to switch an illumination state of the light source at a flashing frequency.

The flashing frequency may be in the range defined by 1 and 200 Hz, preferably by 1 and 150 Hz, further preferably by 1 and 100 Hz.

The illumination element may comprise an identification tag. The identification tag may, in particular, comprise any of an RFID, an NFC, or any other suitable tag. The identification tag may be of advantage in automatic identification of the illumination element via radio-wave transmission, for example. For example, the identification tag may comprise an RFID tag configured to store a unique identifier. The unique identifier may be generated/updated, for example, when a new battery is installed in the illumination element. The unique identifier may then be of advantage, for example, in tracking a life cycle of the battery of the illumination element. The unique identifier may, generally, allow identification of the illumination element. For example, the battery installation time may be stored in a database together with the unique identifier, instead.

The identification tag may comprise an active identification tag. Preferably, however, the identification tag may comprise a passive identification tag. An active identification tag may be understood to comprise a tag that may emit a signal of its own accord such as an active RFID tag. A passive identification tag may, on the other hand, only emit a signal in response to receiving a stimulating signal such as a passive RFID tag. An advantage of using a passive identification tag may be reduction in consumption of energy by the illumination element.

The illumination element may comprise a heat exchanger configured to exchange heat with the reaction medium.

The illumination element may comprise a passive heat exchanger.

The illumination element may comprise an active heat exchanger. The active heat exchanger may, for example, comprise a pump, a circuit with a circulating (or working) fluid, and two or more heat exchanging surfaces. At least one of the two or more heat exchanging surfaces may, for example, be configured to exchange heat with a heat source, while at least one other of the two or more heat exchanging surfaces may be configured to exchange heat with a heat sink. Thus, heat may be transferred between the heat source and the heat sink. The heat source may comprise, for example, the illumination element, or at least a part thereof.

The heat exchanger may be configured to regulate a temperature of the illumination element. Regulating the temperature of the illumination element may be of particular advantage as a variation in temperature may affect the growth of algae in the reaction medium.

The control unit may be further configured to control the heat exchanger.

The temperature sensor may be configured to sense the temperature in an interior of the illumination element, and wherein the heat exchanger may be configured to regulate the temperature based, at least in part, on the temperature sensed by the temperature sensor.

According to a second aspect the present invention relates to a supply station for supplying energy to an illumination element, the supply station configured to be connected to the illumination element.

The illumination element may comprise an illumination element as described above.

The supply station may comprise a magnet configured to attract the illumination element. The magnet may, preferably, comprise an electromagnet.

The supply station may comprise a supply station housing.

The supply station housing may comprise a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material. The material can be rigid or flexible (inflatable/expandable/stretchable).

The supply station may be configured to float on top of the container. This configuration may be advantageous as illumination elements may simply float up to reach the supply station. For example, illumination elements may be configured to have a density similar to that of the reaction medium in an initial stage of cultivation. As algae grows in the reaction medium, the density of the illumination element becomes lower than that of the reaction medium (in absence of any control by the density control unit), and the illumination element is naturally pushed up.

The supply station may be configured to be installed inside the container.

The supply station may be configured to be installed at a bottom of the container. This configuration may be advantageous as illumination elements may simply sink down to reach the supply station. As described above, in absence of density regulation, the illumination element may naturally float up. However, as also described above, the illumination element may be configured to draw fluid in. Thus, the illumination element may also sink down simply.

The supply station may be configured to be, at least partially, immersed in a reaction medium of a container.

The supply station housing is, at least partially, impermeable and/or inert to the reaction medium.

The supply station may be configured to supply energy to the illumination element. The supply station may, in particular, charge the battery of the illumination element.

The supply station may be configured to receive energy from a local grid.

The supply station may comprise a plug to allow receiving energy from the local grid. The local grid may supply energy generated from any source, including renewable sources of energy such as solar energy, wind energy, energy from biomass, biogas, etc. The local grid may be understood to comprise not only energy generated remote from a location of the container, but also energy generated locally by means, for example, of solar cells on a roof of a building where the container is housed.

The supply station may comprise a supply station battery.

The supply station battery may be rechargeable. As described later, a supply station battery may be of particular advantage in improving ease-of-installation and use of a system according to the present invention.

The supply station may be configured to charge the supply station battery using the energy from the local grid.

The supply station may be configured to supply energy to the illumination element by means of the supply station battery.

The supply station may comprise a supply terminal.

The supply terminal may be configured to be electrically connected to the local grid.

The illumination element may be configured to be connected to the supply terminal.

The supply station may be configured to supply energy to the illumination element through the supply terminal. In other words, the supply station may supply energy to the illumination element by means of a wired connection.

The supply terminal may comprise a supply station induction coil.

The supply station may be configured to drive alternating current from the local grid through the supply station induction coil.

The supply terminal may comprise a socket, wherein the socket may be complementary to a plug of the illumination element as described above.

The supply station may comprise a plurality of supply terminals.

The supply station may comprise a supply station communication unit.

The supply station communication unit may be configured to receive data from a communication unit of the illumination element as described above.

The supply station communication unit may be configured to send data to and/or receive data from an external device.

The supply station may comprise a supply station data processing unit and/or a supply station memory unit.

The supply station communication unit may be configured to communicate with the supply station data processing unit and/or with the supply station memory unit.

A storage capacity of the supply station battery may be in the range defined by 0.050 and 5000 kWh, preferably by 1 and 500 kWh, further preferably by 5 and 100 kWh.

The supply station battery may comprise any of a Li-ion battery, a Li-polymer battery, a solid-state battery, a super capacitor battery, an Ni-Cd battery, an Ni-Metal Hydride battery, a redox flow battery, a sodium ion battery, and a lithium-sulphur solid-state battery.

The magnet of the supply station as described above may comprise an electromagnet.

The supply station may comprise a magnet control unit, wherein the magnet control unit may be configured to activate/deactivate the electromagnet. The magnet control unit may be further configured to control a strength and/or a spatial profile of an electromagnetic field generated by the electromagnet. Control of the strength and/or the spatial profile may be of particular advantage in allowing position of the illumination element within a reaction medium of the container, as described further below.

The supply station communication unit and/or the supply station data processing unit may be configured to communicate with the magnet control unit.

The supply station may be further configured to supply an additive to the illumination element.

The supply station may be configured to receive a sample for analysis from the illumination element.

The communication unit of an illumination element as described above may be configured to communicate with the supply station communication unit as described above.

According to a third aspect, the present invention relates to a system for illuminating an interior of a container, the system comprising: a plurality of illumination elements, wherein each of the plurality of illumination elements is an illumination element as described above.

The system may comprise a supply station as described above.

The system may comprise a system data processing unit.

The system may comprise a system communication unit.

The system communication unit may be configured to communicate with the system data processing unit.

The system communication unit may be configured to communicate with the supply station communication unit and/or the communication unit of the illumination element as described above. Thus, embodiments of the present technology may allow data captured *in-situ* by, for example, an illumination element immersed in a reaction medium of the container to be received and processed remotely. The remote processing may allow more complex analyses to be carried out as greater processing capacity may be available than in a data processing unit of the illumination element.

At least two of the plurality of illumination elements may comprise illumination elements as described above, wherein the communication unit of one of the at least two illumination elements may be configured to communicate with the communication unit of the other of the at least two illumination elements. For example, data from the sensors comprised in the illumination element as described above may be used to obtain positional information within the reaction medium such as a density of algae, a pH level, a COz concentration, etc. The data may be used, for example, to determine a wavelength of light to be emitted by the light source of an illumination element at a defined position in the reaction volume. Or, for example, the data may be used to determine if more illumination elements need to be added in a defined region of the reaction medium.

According to a fourth aspect, the present invention relates to a use of an in illumination element as described above in a photobioreactor or a use of a system as described above in a photobioreactor.

The photobioreactor may comprise the container and the container may comprise a reaction medium.

Generally, it will be understood that the photobioreactor may thus comprise the container, the reaction medium and at least one illumination element (and preferably a plurality of illumination elements).

The container may comprise a volume at least as large as 0.1 m³, preferably at least as large as 0.5 m³, further preferably at least as large as 1 m³.

The use as described above, wherein the illumination element may comprise a housing made of metal.

The photobioreactor may be used for algae cultivation, cultivation of phototrophic organisms, cyanobacteria, corals, underwater plants, moss, microalgae, macroalgae, artificial photosynthetic systems, or biomass production. The algae may comprise any of Chlorella vulgaris, Spirulina platensis, Haematococcus pluvialis, Phaeodactylum tricornutum, Nostoc muscorum, Chlamydomonas reinhardtii, Dunaliella salina, Nannochloropsis, Scenedesmus, Botryococcus braunii, Porphyridium, Synechocystis, Synechococcus, and co-cultures of phototrophic and non-phototrophic organisms.

The container may comprise an agitator, such as a pump, a flow pump, a gas circulator, a gas inlet, or a gas outlet.

The container may comprise at least one metal appliance.

According to a fifth aspect, the present invention relates to a method for illuminating an interior of a container, the method comprising providing an illumination element as described above.

The method may comprise providing a plurality of illumination elements, wherein each of the plurality of illumination elements may be an illumination element as described above.

The method may comprise providing a supply station for supplying energy to the illumination element, wherein the supply station may comprise a supply station as described above.

The method may comprise changing a density of the illumination element.

The density may be changed by at most 25%, preferably by at most 20%, further preferably by at most 15%.

The method may comprise changing a density of a plurality of the plurality of illumination elements.

The method may comprise arranging the plurality of illumination elements in a volume of the container with, at least significantly, a defined number density.

The container may comprise a reaction medium, wherein the defined number density may be based, at least in part, on a total illuminated surface area per unit volume of the reaction medium.

The total illuminated surface area may be in the range defined by 1 and 30 m², preferably by 1 and 25 m², further preferably by 1 and 20 m², for 1000 L volume of the reaction medium.

The method may comprise changing the number density of the plurality of illumination elements.

The method may comprise changing the number density based, at least in part, on a total illuminated surface area per unit volume of the reaction medium.

The method may comprise changing the total illuminated surface area per unit volume of the reaction medium by a factor less than 30, preferably less than 25, further preferably less than 20.

The method may comprise maintaining the number density constant for at least 10 minutes, preferably at least 30 minutes, further preferably at least 1 hour and then changing the number density. Note, however, that keeping the number density constant or with defined changes over time periods may depend on the organism to be cultivated in the photobioreactor, its growth curve, or its growth characteristics. Typical algae, for example, have doubling times in the range 1 day to 14 days, fast growing algae have doubling times in the range of 1 h to 24 h, and genetically modified algae can have even faster doubling times of less than 1 hour.

The container may be used for algae cultivation, and the method may comprise changing the number density based, at least in part, on a level of growth of the algae in the container. The level of growth may be determined based, for example, on a progress of the growth curve of the algae or on the level of product formation of a value-added product as described above.

The container may be used for algae cultivation, wherein the method may comprise changing the number density based, at least in part, on a type of the algae.

The container may comprise a reaction medium, and wherein the method may comprise immersing the illumination element in the reaction medium.

The method may comprise retrieving an illumination element immersed in a reaction medium of the container.

The method may comprise cleaning the illumination element, or at least a part thereof.

The method may comprise controlling a wavelength of light emitted by the illumination element.

The method may comprise controlling a power at which light is emitted by the illumination element.

The method may comprise controlling the wavelength of and/or the power at which light is emitted by the illumination element based, at least in part, on data received from a sensor of the illumination element.

The method may comprise detecting a low remaining battery capacity of an illumination element. The method may comprise retrieving the illumination element and recharging the battery thereof.

The method may comprise immersing the illumination element with the recharged battery in the reaction medium of the container.

The method may comprise immersing an illumination element with a charged battery to replace the illumination element with the low remaining battery capacity.

The method may comprise wrapping the illumination element in a protective film before immersing in the reaction medium.

The protective film may, at least partially, be impermeable to the reaction medium.

The protective film may, at least in part, be inert to the reaction medium.

Cleaning the illumination element, or at least a part thereof, may comprise changing the protective film. Thus, the illumination element may be cleaned easily and simply.

The method may comprise moving the illumination element within the reaction medium through standard techniques of underwater movement.

The system as described above may be used to perform the method as described above.

The illumination element as described above may be used in the use as described above and/or in the method as described above.

The present invention is also described by the following numbered embodiments.

Below illumination element embodiments will be discussed. These are abbreviated by the letter "I" followed by a number. Whenever reference is herein made to the illumination element embodiments, the following embodiments are meant.
I1. An illumination element for illuminating an interior of a container, wherein the illumination element comprises:
   a light source, and
   a battery configured to supply energy to the light source.
I2. The illumination element according to the preceding embodiment, wherein the illumination element comprises a plurality of light sources.
I3. The illumination element according to the preceding embodiment, wherein a wavelength of light emitted by a first of the plurality of light sources is different from a wavelength of light emitted by a second of the plurality of light sources.
I4. The illumination element according to any of the preceding illumination element embodiments but without the features of the preceding embodiment, wherein a wavelength of light emitted by any one of the plurality of light sources is, at least significantly, identical to a wavelength of light emitted by any other of the plurality of light sources.
I5. The illumination element according to any of the preceding illumination element embodiments, wherein the illumination element is configured to be connected to a supply station.
   It will be understood that a connection may in particular refer to an electrical connection to transfer energy.
I6. The illumination element according to any of the preceding illumination element embodiments, wherein the illumination element is configured to be attracted and/or repelled by a supply station, and wherein the illumination element comprises an attachment element configured to allow the illumination element to be attracted and/or repelled by the supply station.
I7. The illumination element according to the preceding embodiment, wherein the attachment element comprises a magnetic material.
I8. The illumination element according to any of the preceding illumination element embodiments, wherein the battery is a rechargeable battery.
I9. The illumination element according to the preceding embodiment and with the features of embodiment I5, wherein the battery is configured to receive energy from the supply station.
I10. The illumination element according to any of the preceding illumination element embodiments, wherein the battery comprises any of a Li-ion battery, a Li-polymer battery, a solid-state battery, a super capacitor battery, an Ni-Cd battery, an Ni-Metal Hydride battery, a redox flow battery, a sodium ion battery, and/or a lithium-sulphur solid-state battery.
I11. The illumination element according to any of the 2 preceding embodiments, wherein the energy is received wirelessly.
I12. The illumination element according to any of the 3 preceding embodiments, wherein the energy is received inductively.
I13. The illumination element according to the preceding embodiment, wherein the illumination element comprises an illumination element induction coil.
I14. The illumination element according to any of the 3 preceding embodiments, wherein the battery is configured to receive energy from the supply station in a configuration such that a minimum distance between the illumination element, or at least a part thereof, and the supply station, or at least a part thereof, is less than 1 m, preferably less than 10 cm, further preferably less than 1 cm.
I15. The illumination element according to any of the 3 preceding embodiments, wherein a rate at which energy is received inductively is at least 1 W, preferably at least 10 W, further preferably at least 100 W.
I16. The illumination element according to any of the preceding illumination element embodiments, wherein the illumination element comprises a rectifier configured to convert an alternating current to a direct current.
I17. The illumination element according to the preceding embodiment and with the features of embodiment I13, wherein the rectifier is connected to the illumination element induction coil.
I18. The illumination element according to any of the 2 preceding embodiments, wherein the rectifier is connected to the battery such that the direct current can be used to charge the battery.
I19. The illumination element according to any of the preceding illumination element embodiments and with the features of embodiment I9 but without the features of embodiment I11, wherein the energy is received over a wired connection.
I20. The illumination element according to the preceding embodiment and with the features of embodiment I5, wherein the illumination element comprises a plug complementary to a socket of the supply station.
   The rectifier, as described above, may be connected between the plug and the battery, thus allowing the illumination element to be charged by means of an alternating current as provided, for example, by a local grid.
I21. The illumination element according to any of the preceding embodiments, wherein the illumination element comprises a housing.
I22. The illumination element according to the preceding embodiment, wherein the housing comprises any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material.
   The material can be rigid or flexible (inflatable/expandable/stretchable).
I23. The illumination element according to any of the 2 preceding embodiments, wherein the housing comprises a plurality of housing sections, such as two housing sections.
I24. The illumination element according to the preceding embodiment, wherein the two housing sections are configured to be releasably attached to each other.
I25. The illumination element according to the preceding embodiment, wherein the two housing sections are configured to be attached to each other by means of a thread connection.
I26. The illumination element according to any of the 2 preceding embodiments, wherein the two housing sections are configured to be attached to each other by means of a bayonet connection.
I27. The illumination element according to any of the preceding illumination element embodiments and with the features of embodiment I21, wherein the housing comprises, at least significantly, any of a spherical, an oval, a cylindrical, an angular, a polygonal, or a platonic solid shape.
I28. The illumination element according to the preceding embodiment and with the features of embodiment I23, wherein each of the two housing sections comprises, at least significantly, a hemispherical shape.
I29. The illumination element according to any of the preceding illumination element embodiments and with the features of embodiment I21, wherein the housing is, at least partially, transparent to the light emitted by the light source.
I30. The illumination element according to any of the preceding illumination element embodiments, wherein the container comprises a reaction medium, and wherein the illumination element is configured to be, at least partially, submerged in the reaction medium.
I31. The illumination element according to the preceding embodiment and with the features of embodiment I21, wherein the housing is, at least partially, impermeable to the reaction medium.
I32. The illumination element according to any of the preceding illumination element embodiments and with the features of embodiment I21, wherein the light source is embedded in the housing.
I33. The illumination element according to any of the preceding illumination element embodiments and with the features of embodiment I21 but without the features of the preceding embodiment, wherein the light source is arranged within the housing.
I34. The illumination element according to any of the preceding illumination element embodiments and with the features of embodiment I21 but without the features of any of the 2 preceding embodiments, wherein the light source is arranged on an exterior of the housing.
I35. The illumination element according to the preceding embodiment, wherein the container comprises a reaction medium, and wherein the light source is, at least partially, inert and/or impermeable to the reaction medium.
I36. The illumination element according to any of the preceding illumination element embodiments, wherein the illumination element comprises a data processing unit.
I37. The illumination element according to any of the preceding illumination element embodiments, wherein the illumination element comprises a control unit, wherein the control unit is configured to control the light source.
   Controlling the light source may be understood to comprise controlling a state of the light source, i.e., switching the light source on or off. Controlling may also be understood to comprise controlling a wavelength and/or an intensity and/or a flashing frequency of the light source.
I38. The illumination element according to the preceding embodiment and with the features of embodiment I2, wherein the control unit is configured to control each of the plurality of light sources.
I39. The illumination element according to any of the 2 preceding embodiments and with the features of embodiment I36, wherein the control unit is configured to receive data from the data processing unit.
I40. The illumination element according to the preceding embodiment, wherein the control unit is configured to control the light source based, at least in part, on the data received from the data processing unit.
I41. The illumination element according to any of the preceding illumination element embodiments, wherein the illumination element further comprises a communication unit configured to send data to and/or receive data from an external device and/or element.
I42. The illumination element according to the preceding embodiment, wherein the communication unit is configured to send the data wirelessly.
I43. The illumination element according to any of the 2 preceding embodiments and with the features of embodiment I36, wherein the communication unit is configured to receive data from the data processing unit.
I44. The illumination element according to any of the 3 preceding embodiments and with the features of embodiment I36, wherein the communication unit is configured to send data to the data processing unit.
I45. The illumination element according to any of the preceding illumination element embodiments, wherein the illumination element further comprises a sensor.
I46. The illumination element according to the preceding embodiment and with the features of embodiment I36, wherein the sensor is configured to send data to the data processing unit.
I47. The illumination element according to any of the preceding illumination element embodiments and with the features of embodiment I45, wherein the sensor comprises a position sensor and/or an orientation sensor.
I48. The illumination element according to any of the preceding illumination element embodiments and with the features of embodiment I45, wherein the sensor comprises a temperature sensor.
I49. The illumination element according to the preceding embodiment, wherein the temperature sensor is configured to measure a temperature within the illumination element.
I50. The illumination element according to any of the 2 preceding embodiments, wherein the temperature sensor is configured to measure a temperature of an environment of the illumination element.
I51. The illumination element according to any of the preceding illumination element embodiments and with the features of embodiment I45, wherein the sensor comprises a pH sensor.
I52. The illumination element according to any of the preceding illumination element embodiments and with the features of embodiment I45, wherein the sensor comprises a pressure sensor.
I53. The illumination element according to any of the preceding illumination element embodiments and with the features of embodiment I45, wherein the sensor comprises a COz concentration sensor.
I54. The illumination element according to any of the preceding illumination element embodiments and with the features of embodiment I45, wherein the sensor comprises a brightness sensor.
I55. The illumination element according to any of the preceding illumination element embodiments and with the features of embodiment I45, wherein the sensor comprises at least one of a(n): O₂ concentration sensor, viscosity sensor, turbidity sensor, density sensor, conductivity sensor, impedance sensor, optical density sensor, photosynthetically active radiation (PAR) sensor, camera, microscope, IR/Raman-spectroscopy-sensor, cell potential sensor, potentiostat sensor, cell cytometry sensor, specific ion-sensor (e. g., Na⁺, Cl⁻), specific nutrient concentrations sensor, photometer sensor, proximity sensor (to detect a wall of the container), and proximity sensor / distance sensor between two illumination elements.
I56. The illumination element according to any of the preceding illumination element embodiments and with the features of embodiment I45, wherein the sensor comprises a battery capacity sensor configured to measure a remaining capacity of the battery.
I57. The illumination element according to the preceding embodiment and with the features of embodiments I43, and I46, wherein the communication unit is configured to send data related, at least in part, to the remaining capacity of the battery.
I58. The illumination element according to any of the preceding illumination element embodiments and with the features of embodiments I36, and I45, wherein the data processing unit comprises a sensor threshold associated with the sensor.
I59. The illumination element according to the preceding embodiment and with the features of embodiments I43, and I46, wherein the data processing unit is configured, in response to the data received from the sensor crossing the sensor threshold, to send to the communication unit data related, at least in part, thereto.
I60. The illumination element according to any of the preceding illumination embodiments and with the features of embodiments I21, and I45, wherein the sensor is configured to detect a radiative signal from an environment of the illumination element, and wherein the housing is, at least partially, transparent to the radiative signal.
I61. The illumination element according to any of the preceding illumination element embodiments and with the features of embodiment I30, wherein a density of the illumination element is, at least significantly, similar to a density of the reaction medium, or at least a part thereof.
I62. The illumination element according to any of the preceding illumination element embodiments, wherein the illumination element comprises a density control unit configured to control a density of the illumination element.
I63. The illumination element according to the preceding embodiment and with the features of embodiment I36, wherein the density control unit is configured to receive data from the data processing unit.
I64. The illumination element according to the preceding embodiment, wherein the density control unit is configured to control the density based, at least in part, on the data received from the data processing unit.
I65. The illumination element according to the preceding embodiment and with the features of embodiment I46, wherein the density control unit is configured to control the density based, at least in part, on the data received from the sensor.
I66. The illumination element according to any of the preceding illumination element embodiments and with the features of embodiments I61, and I62, wherein the density control unit is configured to control the density to within 25%, preferably within 20%, further preferably within 15%, of the density of the reaction medium.
I67. The illumination element according to any of the preceding illumination element embodiments and with the features of embodiment I62, wherein the density control unit comprises a pump.
I68. The illumination element according to any of the preceding illumination element embodiments and with the features of embodiment I62, wherein the density control unit comprises a membrane.
I69. The illumination element according to the preceding embodiment, wherein the membrane comprises a flexible material.
I70. The illumination element according to any of the preceding illumination element embodiments and with the features of embodiment I62, wherein the density control unit comprises a movable cylinder.
I71. The illumination element according to any of the preceding illumination element embodiments and with the features of embodiment I62, wherein the density control unit comprises a storage tank configured to store a fluid.
I72. The illumination element according to the preceding embodiment and with the features of embodiment I67, wherein the density control unit is configured to draw liquid into the storage tank.
I73. The illumination element according to the preceding embodiment and with the features of embodiment I67, wherein the density control unit is configured to pump liquid out of the storage tank.
I74. The illumination element according to any of the preceding illumination element embodiments, wherein the container comprises a reaction medium, and wherein the illumination element is configured to introduce an additive into the reaction medium.
I75. The illumination element according to the preceding embodiment, wherein the additive comprises at least one of a gas, a nutrient, or a chemical.
I76. The illumination element according to any of the 2 preceding embodiments, wherein the additive comprises any of CO₂, O₃, and H₂O₂.
I77. The illumination element according to any of the preceding illumination element embodiments, wherein the illumination element comprises a sampling unit configured to facilitate sample analysis.
I78. The illumination element according to the preceding embodiment, wherein the container comprises a reaction medium, and wherein the illumination element, particularly the sampling unit thereof, is configured to draw and store a sample from the reaction medium.
I79. The illumination element according to any of the 2 preceding embodiments, wherein the illumination element, particularly the sampling unit thereof, is configured to release a stored sample into a supply station.
I80. The illumination element according to any of the preceding illumination element embodiments, wherein the battery comprises a storage capacity in the range defined by 0.005 and 50 kWh per m² of illuminated surface area of the illumination element, preferably by 0.1 and 25 kWh per m², further preferably by 1 and 10 kWh per m².
I81. The illumination element according to any of the preceding illumination element embodiments and with the features of embodiment I2, wherein a wavelength of at least one of the plurality of light sources is in the range defined by 150 and 450 nm, preferably by 180 and 400 nm, further preferably by 200 and 380 nm.
I82. The illumination element according to any of the preceding illumination element embodiments and with the features of embodiment I2, wherein a wavelength of at least one of the plurality of light sources is in the range defined by 350 and 900 nm, preferably by 370 and 850 nm, further preferably by 380 and 800 nm.
I83. The illumination element according to any of the preceding illumination element embodiments and with the features of embodiment I2, wherein a wavelength of at least one of the plurality of light sources is in the range defined by 700 and 1200 nm, preferably by 750 and 1100 nm, further preferably by 800 and 1000 nm.
I84. The illumination element according to any of the preceding illumination element embodiments, wherein the light source is configured to emit light with a light intensity generated on the surface of the illumination element in the range defined by of 1 and 5000 µmol/m²/s, preferably by 2 and 4000 µmol/m²/s, further preferably by 5 and 3000 µmol/m²/s.
I85. The illumination element according to any of the preceding illumination element embodiments, wherein the light source comprises a light emitting diode (LED).
I86. The illumination element according to the preceding embodiment, wherein the LED comprises an organic LED (OLED).
I87. The illumination element according to any of the preceding embodiments, wherein the light source comprises a quantum dot.
I88. The illumination element according to any of the 3 preceding embodiments, wherein an efficiency of the LED is at least 10%, preferably at least 30%, further preferably at least 50%.
I89. The illumination element according to any of the preceding illumination embodiments, wherein the light source comprises a laser such as an organic solid state laser.
I90. The illumination element according to any of the preceding illumination element embodiments, wherein the container is used for cultivation of algae, and wherein a volume of the illumination element is based, at least in part, on a density and/or type of the algae.
I91. The illumination element according to any of the preceding illumination element embodiments, wherein a volume of illumination element is based, at least in part, on a number density of illumination elements to be used in the container.
I92. The illumination element according to any of the preceding illumination element embodiments, wherein a volume of the illumination element is in the range defined by 100 and 600,000 cm³, preferably by 250 and 30,000 cm³, further preferably by 500 and 15,000 cm³.
I93. The illumination element according to any of the preceding illumination element embodiments, wherein the illumination element is configured to fluoresce.
I94. The illumination element according to the preceding embodiment, wherein the illumination element comprises a fluorescent tag, the fluorescent tag comprising fluorescent paint.
I95. The illumination element according to any of the preceding illumination element embodiments and with the features of embodiment I21, wherein the housing comprises an external surface, the external surface in contact with a reaction medium of the container, and an internal surface opposite the external surface.
I96. The illumination element according to the preceding embodiment and with the features of embodiment I94, wherein the fluorescent tag is attached to the external surface.
I97. The illumination element according to any of the preceding illumination element embodiments and with the features of embodiment I37, wherein the control unit is configured to switch an illumination state of the light source at a flashing frequency.
I98. The illumination element according to the preceding embodiment, wherein the flashing frequency is in the range defined by 1 and 200 Hz, preferably by 1 and 150 Hz, further preferably by 1 and 100 Hz.
I99. The illumination element according to any of the preceding illumination element embodiments, wherein the illumination element comprises an identification tag.
I100. The illumination element according to the preceding embodiment, wherein the identification tag comprises an active identification tag.
I101. The illumination element according to the penultimate embodiment, wherein the identification tag comprises a passive identification tag.
I102. The illumination element according to any of the preceding illumination element embodiments and with the features of embodiment I30, wherein the illumination element comprises a heat exchanger configured to exchange heat with the reaction medium.
I103. The illumination element according to the preceding embodiment, wherein the illumination element comprises a passive heat exchanger.
I104. The illumination element according to any of the 2 preceding embodiments, wherein the illumination element comprises an active heat exchanger.
I105. The illumination element according to any of the 2 preceding embodiments, wherein the heat exchanger is configured to regulate a temperature of the illumination element.
I106. The illumination element according to the preceding embodiment and with the features of embodiments I37, and I104, wherein the control unit is further configured to control the heat exchanger.
I107. The illumination element according to any of the 2 preceding embodiments and with the features of embodiment I48, wherein the temperature sensor is configured to sense the temperature in an interior of the illumination element, and wherein the heat exchanger is configured to regulate the temperature based, at least in part, on the temperature sensed by the temperature sensor.
   Below supply station embodiments will be discussed. These are abbreviated by the letter "D" followed by a number. Whenever reference is herein made to the supply station embodiments, the following embodiments are meant.
D1. A supply station for supplying energy to an illumination element, wherein the supply station is configured to be connected to the illumination element.
D2. The supply station according to the preceding embodiment, wherein the illumination element is an illumination element according to any of the preceding illumination element embodiments.
D3. The supply station according to any of the preceding supply station embodiments, wherein the supply station comprises a supply station magnet configured to attract and/or repel the illumination element.
D4. The supply station according to any of the preceding supply station embodiments, wherein the supply station comprises a supply station housing.
D5. The supply station according to the preceding embodiment, wherein the supply station housing comprises a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material. The material can be rigid or flexible (inflatable/expandable/stretchable).
D6. The supply station according to any of the preceding supply station embodiments, wherein the supply station is configured to float on top of the container.
D7. The supply station according to any of the preceding supply station embodiments, wherein the supply station is configured to be installed inside the container.
D8. The supply station according to the preceding embodiment, wherein the supply station is configured to be installed at a bottom of the container.
D9. The supply station according to any of the preceding supply station embodiments, wherein the supply station is configured to be, at least partially, immersed in a reaction medium of a container.
D10. The supply station according to the preceding embodiment and with the features of embodiment D4, wherein the supply station housing is, at least partially, impermeable and/or inert to the reaction medium.
D11. The supply station according to any of the preceding supply station embodiments, wherein the supply station is configured to supply energy to the illumination element.
D12. The supply station according to any of the preceding supply station embodiments, wherein the supply station is configured to receive energy from a local grid.
D13. The supply station according to the preceding embodiment, wherein the supply station comprises a plug to allow receiving energy from the local grid.
D14. The supply station according to any of the preceding supply station embodiments, wherein the supply station comprises a supply station battery.
D15. The supply station according to the preceding embodiment, wherein the supply station battery is rechargeable.
D16. The supply station according to the preceding embodiment and with the features of embodiment D12, wherein the supply station is configured to charge the supply station battery using the energy from the local grid.
D17. The supply station according to any of the 3 preceding embodiments and with the features of embodiment D11, wherein the supply station is configured to supply energy to the illumination element by means of the supply station battery.
D18. The supply station according to any of the preceding supply station embodiments, wherein the supply station comprises a supply terminal.
D19. The supply station according to the preceding embodiment and with the features of embodiment D12, wherein the supply terminal is configured to be electrically connected to the local grid.
D20. The supply station according to any of the preceding supply station embodiments and with the features of embodiment D18, wherein the illumination element is configured to be connected to the supply terminal.
D21. The supply station according to the preceding embodiment and with the features of embodiment D12, wherein the supply station is configured to supply energy to the illumination element through the supply terminal.
D22. The supply station according to any of the preceding supply station embodiments and with the features of embodiment D18, wherein the supply terminal comprises a supply station induction coil.
D23. The supply station according to the preceding embodiment and with the features of embodiment D19, wherein the supply station is configured to drive alternating current from the local grid through the supply station induction coil.
D24. The supply station according to any of the preceding supply station embodiments and with the features of embodiment D18, wherein the supply terminal comprises a socket.
D25. The supply station according to the preceding embodiment, wherein the illumination element is an illumination element with the features of embodiment I20, wherein the socket is complementary to the plug of the illumination element.
D26. The supply station according to any of the preceding supply station embodiments and with the features of embodiment D18, wherein the supply station comprises a plurality of supply terminals.
D27. The supply station according to any of the preceding supply station embodiments, wherein the supply station comprises a supply station communication unit.
D28. The supply station according to the preceding embodiment, wherein the illumination element comprises an illumination element with the features of embodiment I41, and wherein the supply station communication unit is configured to receive data from the communication unit of the illumination element.
D29. The supply station according to any of the 2 preceding embodiments, wherein the supply station communication unit is configured to send data to and/or receive data from an external device.
D30. The supply station according to any of the preceding supply station embodiments, wherein the supply station comprises a supply station data processing unit and/or a supply station memory unit.
D31. The supply station according to the preceding embodiment and with the features of embodiment D27, wherein the supply station communication unit is configured to communicate with the supply station data processing unit and/or with the supply station memory unit.
D32. The supply station according to any of the preceding supply station embodiments and with the features of embodiment D14, wherein a storage capacity of the supply station battery is in the range defined by 0.050 and 5000 kWh, preferably by range 1 and 500 kWh, further preferably by 5 and 100 kWh.
D33. The supply station according to any of the preceding supply station embodiments and with the features of embodiment D14, wherein the supply station battery comprises any of a Li-ion battery, a Li-polymer battery, a solid-state battery, a super capacitor battery, an Ni-Cd battery, an Ni-Metal Hydride battery, a redox flow battery, a sodium ion battery, and a lithium-sulphur solid-state battery.
D34. The supply station according to any of the preceding supply station embodiments and with the features of embodiment D3, wherein the supply station magnet comprises an electromagnet.
D35. The supply station according to the preceding embodiment, wherein the supply station comprises a magnet control unit, wherein the magnet control unit is configured to activate/deactivate the electromagnet.
D36. The supply station according to the preceding embodiment and with the features of any of embodiments D27, and D30, wherein the supply station communication unit and/or the supply station data processing unit is configured to communicate with the magnet control unit.
D37. The supply station according to any of the preceding supply station embodiments, wherein the supply station is further configured to supply an additive to the illumination element.
D38. The supply station according to any of the preceding supply station embodiments, wherein the supply station is configured to receive a sample for analysis from the illumination element.
D39. The supply station according to any of the preceding supply station embodiments and with the features of embodiment D30, wherein the illumination element comprises an illumination element with the features of embodiment I36, wherein a ratio of a processing capacity of the data processing unit of the illumination element to that of the supply station data processing unit is in the range defined by 0.001 and 1, preferably by 0.005 and 1, further preferably by 0.1 and 1.
I108. The illumination element according to any of the preceding illumination element embodiments and with the features of embodiment I41, wherein the communication unit is configured to communicate with a supply station communication unit of a supply station with the features of embodiment D29.
I109. The illumination element according to any of the preceding illumination element embodiments and with the features of embodiment I5, wherein the supply station is a supply station according to any of the preceding supply station embodiments.
I110. The illumination element according to any of the preceding illumination element embodiments and with the features of embodiment I6, wherein the supply station is a supply station according to any of the preceding supply station embodiments with the features of embodiment D3.
   Below system embodiments will be discussed. These are abbreviated by the letter "S" followed by a number. Whenever reference is herein made to the system embodiments, the following embodiments are meant.
S1. A system for illuminating an interior of a container, wherein the system comprises:
   a plurality of illumination elements, wherein each of the plurality of illumination elements is an illumination element according to any of the preceding illumination element embodiments.
S2. The system according to the preceding embodiment, wherein the system further comprises a supply station according to any of the preceding supply station embodiments.
S3. The system according to any of the preceding system embodiments, wherein the system comprises a system data processing unit.
S4. The system according to any of the preceding system embodiments, wherein the system comprises a system communication unit.
S5. The system according to the preceding embodiment and with the features of embodiment S3, wherein the system communication unit is configured to communicate with the system data processing unit.
S6. The system according to any of the preceding system embodiments and with the features of embodiments S2, and S4, wherein the supply station comprises a supply station with the features of embodiment D29, wherein the system communication unit is configured to communicate with the supply station communication unit.
S7. The system according to any of the preceding system embodiments and with the features of embodiment S4, wherein at least one of the plurality of illumination elements comprises an illumination element with the features of embodiment I41, wherein the system communication unit is configured to communicate with the communication unit of the illumination element.
S8. The system according to any of the preceding system embodiments, wherein at least two of the plurality of illumination elements comprise illumination elements each with the features of embodiment I41, wherein the communication unit of one of the at least two illumination elements is configured to communicate with the communication unit of the other of the at least two illumination elements.
   Below use embodiments will be discussed. These are abbreviated by the letter "U" followed by a number. Whenever reference is herein made to the use embodiments, the following embodiments are meant.
U1. Use of an illumination element according to any of the preceding illumination element embodiments or of a system according to any of the preceding system embodiments, in a photobioreactor.
U2. Use according to the preceding embodiment, wherein the photobioreactor comprises the container and the container comprises a reaction medium.
U3. Use according to any of the preceding use embodiments, wherein the container comprises a volume at least as large as 0.1 m³, preferably at least as large as 0.5 m³, further preferably at least as large as 1 m³.
U4. Use according to any of the 3 preceding embodiments, wherein the illumination element comprises a housing made of metal.
U5. Use according to any of the preceding use embodiments, wherein the photobioreactor is used for algae cultivation, cultivation of phototrophic organisms, cyanobacteria, corals, underwater plants, moss, microalgae, macroalgae, artificial photosynthetic systems, or biomass production.
U6. Use according to the preceding embodiment, wherein the algae comprise any of Chlorella vulgaris, Spirulina platensis, Haematococcus pluvialis, Phaeodactylum tricornutum, Nostoc muscorum, Chlamydomonas reinhardtii, Dunaliella salina, Nannochloropsis, Scenedesmus, Botryococcus braunii, Porphyridium, Synechocystis, Synechococcus, and co-cultures of phototrophic and non-phototrophic organisms.
U7. Use according to any of the preceding use embodiments, wherein the container comprises an agitator, such as a pump, a flow pump, or a gas circulator.
U8. Use according to any of the preceding use embodiments, wherein the container comprises at least one metal appliance.
   Below method embodiments will be discussed. These are abbreviated by the letter "M" followed by a number. Whenever reference is herein made to the method embodiments, the following embodiments are meant.
M1. A method for illuminating an interior of a container, wherein the method comprises providing an illumination element according to any of the preceding illumination element embodiments.
M2. The method according to the preceding embodiment, wherein the method comprises providing a plurality of illumination elements, wherein each of the plurality of illumination elements is an illumination element according to any of the preceding illumination element embodiments.
M3. The method according to any of the preceding method embodiments, wherein the method comprises providing a supply station for supplying energy to the illumination element, wherein the supply station comprises a supply station according to any of the preceding supply station embodiments.
M4. The method according to any of the preceding method embodiments, wherein the method comprises changing a density of the illumination element.
M5. The method according to the preceding embodiments, wherein the density is changed by at most 25%, preferably by at most 20%, further preferably by at most 15%.
M6. The method according to any of the preceding method embodiments and with the features of embodiment M2, wherein the method comprises changing a density of a plurality of the plurality of illumination elements.
M7. The method according to any of the preceding method embodiments and with the features of embodiment M2, wherein the method comprises arranging the plurality of illumination elements in a volume of the container with, at least significantly, a defined number density.
M8. The method according to the preceding embodiment, wherein the container comprises a reaction medium, and wherein the defined number density is based, at least in part, on a total illuminated surface area per unit volume of the reaction medium.
M9. The method according to the preceding embodiment, wherein the total illuminated surface area is in the range defined by 1 and 30 m², preferably by 1 and 25 m², further preferably by 1 and 20 m², for 1000 L volume of the reaction medium.
M10. The method according to any of the 2 preceding embodiments, wherein the method comprises changing the number density of the plurality of illumination elements.
M11. The method according to the preceding embodiment, wherein the method comprises changing the number density based, at least in part, on a total illuminated surface area per unit volume of the reaction medium.
M12. The method according to the preceding embodiment, wherein the method comprises changing the total illuminated surface area per unit volume of the reaction medium by a factor less than 30, preferably less than 25, further preferably less than 20.
M13. The method according to any of the 2 preceding embodiments, wherein the method comprises maintaining the number density constant for at least 10 minutes, preferably at least 30 minutes, further preferably at least 1 hour and then changing the number density.
M14. The method according to the preceding embodiment, wherein the container is used for algae cultivation, wherein the method comprises changing the number density based, at least in part, on a level of growth of the algae or product formation in the container.
M15. The method according to the any of the preceding method embodiments with the features of embodiment M10, wherein the container is used for algae cultivation, wherein the method comprises changing the number density based, at least in part, on a type of the algae.
M16. The method according to any of the preceding method embodiments, wherein the container comprises a reaction medium, and wherein the method comprises immersing the illumination element in the reaction medium.
M17. The method according to any of the preceding method embodiments, wherein the method comprises retrieving an illumination element immersed in a reaction medium of the container.
M18. The method according to the preceding embodiment, wherein the method further comprises cleaning the illumination element, or at least a part thereof.
M19. The method according to any of the preceding method embodiments, wherein the method comprises controlling a wavelength of light emitted by the illumination element.
M20. The method according to any of the preceding method embodiments, wherein the method comprises controlling a power at which light is emitted by the illumination element.
M21. The method according to any of the 2 preceding embodiments, wherein the illumination element comprises an illumination element with the features of embodiments I43, and I46, wherein the method comprises controlling the wavelength of and/or the power at which light is emitted by the illumination element based, at least in part, on data received from the sensor of the illumination element.
M22. The method according to any of the preceding method embodiments, wherein the illumination element comprises an illumination element with the features of I57, wherein the method comprises detecting a low remaining battery capacity of the illumination element.
M23. The method according to the preceding embodiment and with the features of embodiment M17, wherein the method comprises retrieving the illumination element and recharging the battery thereof.
M24. The method according to the preceding embodiment, wherein the method further comprises immersing the illumination element with the recharged battery in the reaction medium of the container.
M25. The method according to any of the preceding method embodiments and with the features of embodiment M23 but without the features of the preceding embodiment, wherein the method comprises immersing an illumination element with a charged battery to replace the illumination element with the low remaining battery capacity.
M26. The method according to any of the preceding method embodiments and with the features of embodiment M16, wherein the method comprises wrapping the illumination element in a protective film before immersing in the reaction medium.
M27. The method according to the preceding embodiment, wherein the protective film is, at least partially, impermeable to the reaction medium.
M28. The method according to any of the 2 preceding embodiments, wherein the protective film is, at least in part, inert to the reaction medium.
M29. The method according to any of the 3 preceding embodiments and with the features of embodiment M18, wherein cleaning the illumination element, or at least a part thereof, comprises changing the protective film.
M30. The method according to any of the preceding method embodiments and with the features of embodiment M16, wherein the method comprises moving the illumination element within the reaction medium.
M31. The method according to the preceding embodiment, wherein the method comprises moving the illumination element through any of the standard techniques for effecting underwater movement.
S9. The system according to any of the preceding system embodiments, wherein the system is configured to perform the method according to any of the embodiments M4, M5, M13, M19 to M25, and M30 to M31.
I111. The illumination element according to any of the preceding illumination element embodiments, wherein the illumination element is configured to be used in the use according to any of the preceding use embodiments and/or the method according to any of the preceding method embodiments.

Thus, as described above, a particularly advantageous aspect of the present technology may comprise movable illumination elements (e.g., spheres) within an algae suspension to be cultivated, said movable illumination elements comprising light sources (e.g., LEDs) and other components (e.g., batteries, sensors, additive dispensing mechanisms, etc...). The illumination elements can be removed, added, cleaned, charged, used to add/remove substances/additives, or to measure data (using sensors, as described above) as may be desired/required. The illumination elements may be deliberately moved, controlled, and monitored.

By employing the illumination elements, as described above, even large containers and volumes, for example, may be transformed into photobioreactors using internal lighting.

The illumination elements may be relatively small (compared to a volume of the reaction medium/container) and flexible and may not have to be permanently attached to a container. Thus, maintenance and repair of the illumination system as a whole may be simplified. Further, these features of the illumination elements may allow the realization of an internal illumination in a vessel, tank, basin, or reactor for algae cultivation. This may make it possible to convert vessels, tanks, and containers that may have previously been used for other purposes into photobioreactors in a simple manner. This may be of great potential in algae cultivation.

By varying the number of illumination elements, the amount of light may be varied over the growth cycle of the algae. For example, fewer illumination elements at the beginning when the culture is thin, more illumination elements towards the end when the culture is dense. The illumination elements may be removed from the container as required, making cleaning easy. The illumination elements may be replaced with other illumination elements as required. The new illumination elements may even have different light emissions, depending on the algae's requirements (red, blue light, UV light). Defective illumination elements may be replaced with new illumination elements.

The technical advantages described above may be realized by the illumination elements' design. These illumination elements may be manufactured in large numbers outside of the container/tank, independently of the container/tank, so the advantages and cost savings of series production may be realized. Further technical advantages may be achieved by replacing the illumination elements, which may be small, mobile, and transportable. The technically complex and expensive aspect of overhauling, modernizing, or replacing large containers or equipment may, thus, be reduced.

Advances in LED and battery technology may allow the power and light generation to be packed into the illumination elements without major heat emissions. Highly efficient LEDs such as OLEDs may be particularly suitable.

In other words, embodiments of the present technology may allow the lighting unit to be physically separated from the container, making it technically easier and cheaper to realize scaling. In addition, empty containers may be easily transformed into photobioreactors.

The internal lighting may be generated by relatively small, flexible, and movable illumination elements that may not need to be permanently attached to the container so that they may later be produced (and further developed) independently (of the container) in large series efficiently and economically.

Scaling-up of cultivation volumes in photobioreactors using embodiments of the present invention may allow a linear relationship of the number (and also costs) of the illumination elements. In other words, for example, to scale the production volume by a factor of two, the number of illumination elements may be a factor of two as well, or at least a constant multiple thereof. The linear relationship may be in contrast to conventional photobioreactors with artificial (internal) lighting, as described above, that may require lighting to be installed for the total cultivation volume of all production units present at a production site for maximum lighting. As a production site may typically comprise several photobioreactor units and each unit may be in a different cultivation state not all units may require maximum lighting at once. Thus, more lighting (and, consequently, possibly greater installation cost) has to be installed than may be needed. Embodiments of the present technology may allow installation of only as many illumination elements (and, thus, lighting and costs) as may be needed depending, for example, on average production situation requirements. This may provide an advantageous and risk-based approach for building of large-scale industrial mass production units for algae.

An existing container may be optimized for algae (or, generally, biomass) cultivation and/or growth by replacing the illumination elements. This may significantly improve efficiency, as no structural changes to large installations may be necessary.

Embodiments of the present technology may allow any container and/or tank to be used as an algae reactor, regardless of size (for example, from small garden ponds to 100,000-liter systems). The lighting may be regulated by the size of the illumination elements, the LEDs installed, and their color (for example, different for brown algae and green algae). In this way, different types of algae may be cultivated optimally.

Containers that are temporarily used for other things (e.g., in food or wine production) may be converted into photobioreactors simply by adding the illumination elements. The illumination elements may be easily removed and cleaning them may be simple. Thus, new systems, volumes, and containers may be used for algae cultivation. The simple scaling of photobioreactors may mean that more valuable materials can be produced economically with the help of algae.

Embodiments of the present technology may be employed in a wide range of possible applications, as previously unused containers, vessels, tanks, and reactors may easily be converted into photobioreactors. The present technology may allow cultivating algae in any container, thus enabling the flexible production of algae on an industrial scale. The present technology may be suitable for suitable for closed production facilities or open basins, also outdoors (e.g., pond). Containers that are temporarily not in use may be converted into algae reactors with the help of illumination elements, as described above. It may even be conceivable to cultivate algae in garden ponds or to use them in professional fish farming.

Advantageously, the manufacture and design of the illumination elements may be simple, easy, and efficient. They may be produced in large quantities. The illumination elements may be used in connection with process understanding of the desired algae cultivation. Thus, the present technology may allow the development of precisely fitting illumination elements for the algae, scale, and product desired.

It may also be possible to use the illumination elements as light installations as a design or advertising element in (public) water installations.

Thus, movable illumination elements (e.g., spheres) may be used for internal illumination for the cultivation of phototrophic organisms, in particular microalgae in a liquid culture medium. The one or more illumination elements may be placed in an existing container, vessel, reactor, or basin, thereby enabling the cultivation of phototrophic organisms without other external illumination. Further, the illumination elements may contain one or more light sources (e.g., LED) and a power supply (e.g. rechargeable battery, battery, fuel point, external power supply via cable, etc.) that may supply the light sources with energy.

The illumination elements may be used for the cultivation of phototrophic organisms, in particular prokaryotic and eukaryotic algae, also in combination with other micro- or macro-organisms in a liquid culture medium (e.g., in an aquarium).

The illumination elements may be used for the cultivation of corals in aquaria or to promote growth of corals in natural environments where not enough lighting may be present to prevent dying of corals.

The illumination elements may comprise a translucent material and/or may comprise a coating of a transparent material and may be made of a material inert to the cultivation medium. A shape of the illumination elements can be round, oval, angular, or polygonal. For example, the illumination elements may be spherical in shape and may have a diameter of 40 - 1000 mm (possibly an even larger number).

The light sources may be applied to or incorporated in a transparent or non-transparent material may be inert with respect to the cultivation medium.

The illumination elements may comprise spheres, and may be screwable and may contain threads that make it possible to connect the halves of the sphere to each other.

The illumination elements may comprise a metal plate that may make it possible to remove the illumination elements from the photobioreactor with the aid of a permanent or an electromagnet. The illumination elements may thus be cleaned and / or charged. Charging may take place via induction; the batteries may be replaced or the batteries may be charged via cables.

A light intensity of, for example, 30-3000 µmol/m²/s may be generated at the surface of the illumination elements in the wavelength range, for example, of 200-1000 nm.

The illumination elements may have a density that matches that of the reaction medium. For example, from that of fresh water, *ρ* = 1 g/ml (20°C), up to that of salt water, *ρ* = 1.0857 g/ml (12% salt content, 20°C).

The illumination elements may comprise a mechanism for density adjustment (for example, the density control unit, as described above, that may comprise a movable cylinder or a membrane), that may be used to change a gas volume or pressure in the illumination elements and, thus, the density. This may also enable targeted floating of the illumination elements for removal.

The illumination elements may be modified on the surface in order to achieve better light penetration into the culture medium. The illumination elements may also comprise attachments that may be fitted with a light strip or reflective or fluorescent parts.

The illumination elements may contain one or more waterproof electrical connections via cables, so that the illumination elements may also be charged non-inductively. For example, the cables may comprise suitable plugs such that charging via suitable plugs at a supply station may also be possible. However, charging wirelessly may be preferred.

Some of the illumination elements may also contain substances (additives) that may be helpful for the growth of microorganisms (e.g., nutrients or CO₂ -providing components). These may, then, comprise special additive illumination elements that may be used for dosing nutrients. The additive illumination elements may also comprise light sources.

Some of the illumination elements may contain sensors and measuring devices that may measure, record, and forward data from the reaction medium or culture, as described above. The use of data may make targeted cultivation possible. Data may be transmitted by cable or wirelessly. Wireless transmission of data in liquid media (typically aqueous) may be achieved by means of any suitable technologies that may efficiently transmit signals over short distances (< 100 cm) under water. These may be WLAN frequencies, for example, or acoustic or optical signals. As may be appreciated, optical signals may be such so as to not interfere with the lighting provided by the illumination elements. This can be achieved by using special wavelengths, for example. The illumination elements with sensors may also contain light sources. The illumination elements with sensors may allow external communication (outside the container to a process control system, for example) and/or communication between the illumination elements inside the container. This may make it possible to set up an "illumination element information measurement network" that may enable self-adaptation, self-optimization, and self-control of the process through the use of neural networks and/or artificial intelligence (e.g., illumination elements may swim independently to where they are needed and measure data).

For continuous operation of the illumination elements, it may be necessary to have a charging station/charging device for charging the balls, as well as a device for adding and removing the balls and cleaning them if necessary. These functions may be served, as described above, by a supply station.

The batteries of the illumination elements may be charged at a supply station that may be located outside or inside the container. If it is inside, the supply station may be attached using magnets (for example, to a stainless-steel container) or may be mounted using other attachments (for example, to a plastic container). The supply station may charge the illumination elements, either inductively or by cable. The supply station may also receive signals from the illumination elements with sensors, evaluate them, and forward them to a central control unit. Alternatively, or additionally, the supply station may comprise various sensors to determine the pH value, temperature, oxygen and carbon dioxide concentration, conductivity, and optical density of the reaction medium.

That is, embodiments of the present invention relate to a photobioreactor with internal lighting, where the internal lighting is provided by moving, suspended, or floating elements (e.g., "illumination elements"). The power supply to the light sources inside the illumination elements may be via a rechargeable battery, that may be charged inductively or via cable, as described above. The illumination elements may be configured to have adjustable LED lighting such that a wavelength, an intensity, or a flashing frequency of the lighting may be adjusted.

Further, the illumination elements may be configured for communication between the illumination elements (i.e., between one illumination element and another) and from an external device (such as the supply station as described above) to the illumination elements for measurement, control, and regulation.

The illumination elements may be configured for density changes that may allow the illumination elements to be directed upwards or downwards. The density may be maintained similar to a medium density (of a medium in which the illumination elements are suspended; e.g., ~1 g/ml for fresh water, so that suspended/floating elements are realized).

The size and number of illumination elements may be selected according to tank size, algae density, algae type, etc., as described above.

The illumination elements may be added and removed. The illumination elements may be removed, for example, via a magnet.

Charging of the illumination elements may be achieved outside of the suspension medium either inductively or via cable at a supply station.

Cleaning of the illumination elements may also be carried out outside of the suspension medium, particularly owing to growth of algae in the suspension medium.

The illumination elements may be configured to allow installation of measuring sensors.

Thus, overall, embodiments of the present technology may make it easy to convert large and "arbitrary" containers into photobioreactors with internal lighting.

An advantageous aspect of the present technology may be that no external electric field or emission element for alternating electromagnetic fields may be needed.

The illumination elements according to the present invention may be of particular advantage for algae cultivation. By changing different features of the illumination elements, growth rates of algae may be improved. Particularly relevant for improved cultivation of algae may be the available surface area on the illumination elements, illuminance, and penetration depth. These aspects may be controlled, for example, by controlling a size of the illumination elements, an intensity of the light source within the illumination element, and a number density of illumination elements deployed within a reactor respectively.

Several advantages may be realized by embodiments of the present technology. Firstly, a simple system comprising a supply station and illumination elements for illuminating the interior of a reactor may be provided. Secondly, a number of illumination elements may be increased depending on growth of the algae. Thirdly, a lot of measurement technology may be realized via the illumination elements (for example, optical density through distance between illumination elements and light sensors installed within the illumination elements). Further exemplarily, temperature sensors, light sensors, etc., may be installed. Fourthly, the free-floating illumination elements may enable high flexibility to transform any container and/or tank into a photobioreactor.

As described above, the illumination elements may be charged by means of the supply station. Charging may be possible inductively or via cable. Any number of illumination elements may be flexibly connected to the supply station and separated again. The supply station may be configured to catch the free-floating illumination elements (for charging and/or cleaning). For example, the supply station may comprise one or more magnets for attracting the illumination elements. The supply station may be installed inside the reaction medium and may also be removed from the reaction medium (for cleaning, maintenance, etc.). The power supply via the supply station may be possible with external power (i.e., a local grid) via a cable. The power supply to the light source may be via a rechargeable battery that may be charged outside the reactor. The illumination elements may, for example, have short plug-in connections complementary to sockets on the supply station, or may be charged inductively.

The removal of the illumination elements from the reaction medium may also be of advantage in reducing difficulty and complexity of maintenance of the illumination elements. For example, instead of charging a discharged battery, the battery may simply be replaced. The discharged battery may then be charged later. Or, for example, if the battery, or any other component of the illumination element malfunctions, said battery or component may be easily replaced. The ability to thus replace the battery or the component may be of particular advantage, for example, when the number of illumination elements available is low.

The supply station may be configured to receive signals from the illumination elements. The supply station may further be connected to a computer and may organize the entire process described above.

The illumination elements may comprise various sensors as described above (together with a light source). Alternatively, or additionally, sensors may also be provided on the supply station.

Further, the illumination elements may be configured for providing additives to the reaction medium. In other words, the illumination elements may be configured for dosing, e.g., for CO₂ fumigation (such as a CO₂ - stainless-steel pressure illumination element that may comprise a housing made, at least in part, of stainless steel, and that may comprise pressurized CO₂ that may be released in the reaction medium) or for dosing of nutrients, chemicals, or other additives.

Exemplary features of the invention are further detailed in the figures and the description of the figures below.

### Brief Description of Figures

- Figure 1: depicts a photobioreactor with internal tube lighting;
- Figure 2: depicts an embodiment of a system according to the present invention;
- Figure 3: depicts an embodiment of an illumination element according to the invention;
- Figure 4: depicts a plurality of illumination elements according to the present deployed in a container;
- Figure 5: depicts an embodiment of a supply station according to the present deployed in a container;
- Figure 6: depicts an exemplary arrangement of a plurality of illumination elements a container;
- Figure 7: depicts another exemplary arrangement of a plurality of illumination elements in a container; and
- Figure 8: depicts an embodiment of an illumination element with a heat exchanger.

For the sake of clarity, some features may only be shown in some figures, and others may be omitted. However, also the omitted features may be present, and the depicted and discussed features do not need to be present in all embodiments.

### Detailed Description of Figures

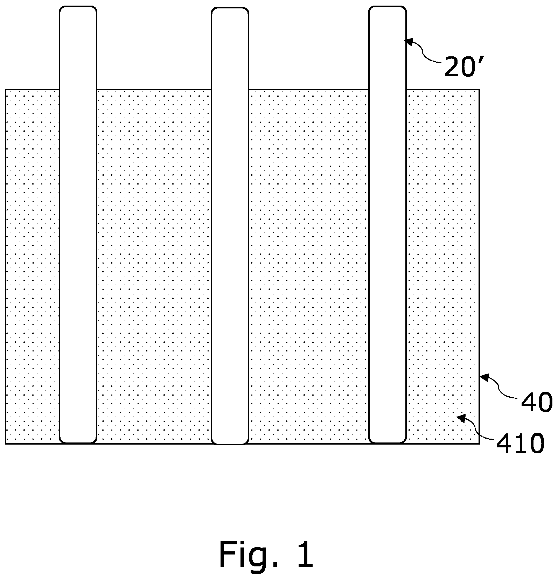

Figure 1 depicts a photobioreactor with internal tube lighting. The photobioreactor may comprise a container 40 comprising a reaction medium 410. One or more lighting tubes 20' may be installed within the container 40 to provide internal lighting. As described above, such a photobioreactor may be subject to certain disadvantages, particularly relating to maintenance and scaling. It is an object of the present invention to provide a photobioreactor that may be easier to maintain and to scale.

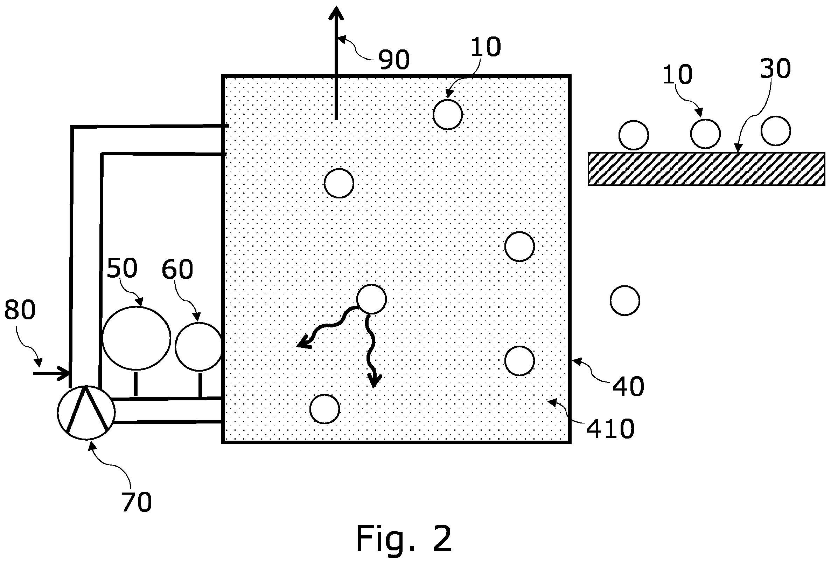

Figure 2 depicts a container 40 comprising a reaction medium 410. Embodiments of the present technology may be of particular relevance for algae cultivation, or more generally, biomass production. Therefore, the reaction medium 410 may comprise strains of an algae to be cultivated. Figure 2 further depicts a system for illuminating an interior of the container 40, the system comprising the plurality of illumination elements 10. The system may further comprise a supply station 30.

The container 40, as depicted in Figure 2, may further comprise the plurality of illumination elements 10, that may be suspended in the reaction medium 410, and that may be used to illuminate the interior of the container 40. The container 40 may, thus, be considered a photobioreactor. According to one aspect, embodiments of the present technology relate to the illumination elements 10.

The supply station 30 may be of advantage in supplying energy to the illumination elements 10, allowing for illumination of the interior of the container 40. According to another aspect, embodiments of the present technology relate to the supply station 30. For example, the illumination element 10 may comprise a rechargeable battery and the supply station 30 may be used to recharge the battery. In particular, as depicted in Figure 2, the supply station 30 may advantageously recharge the battery outside the container 40. This may allow maintenance of the illumination element 10, for example, to be carried out more easily.

The density of the illumination element 10 may be configured to be similar to the density of the reaction medium 410, thus allowing suspension of the illumination element 10 in the reaction medium 410. For example, the reaction medium 410 may have a density of ~ 1 g/mL, corresponding to that of fresh water, and the density of the illumination element 10 may also be, at least significantly, identical to ~ 1 g/mL.

The illumination element 10 is depicted in Figure 2 as comprising a spherical shape. However, generally, the shape of the illumination element 10 may be any of an oval, a cylindrical, an angular, a polygonal, or any other suitable shape, as described above. An advantage of having a spherical shape may be ease of control of the density of the illumination element 10. For example, by changing only one parameter, a diameter of the sphere, the volume and, thus, the density, of the illumination element 10 may be changed. This may be of advantage in controlling the density of the illumination element 10 to be similar to the density of the reaction medium 410. Yet another advantage of a spherical shape may be the facilitation of isotropic emission of light.

Figure 2 depicts other elements that may be relevant for the operation of the photobioreactor 40. For example, a pump 70 may be provided. The pump 70 may be configured to pump COz gas (depicted as the arrow 80) into the container 40. CO₂ may be of advantage in promoting the growth of algae within the container 40. Further, Figure 2 depicts a plurality of sensors, such as a pH-level/O₂ sensor 50, and a temperature sensor 60. These sensors may be employed to monitor changes in the reaction medium 410, thereby monitoring the progress of growth of the algae in the container 40. The growth of algae may be accompanied by expulsion of O₂ gas, as indicated by the arrow 90.

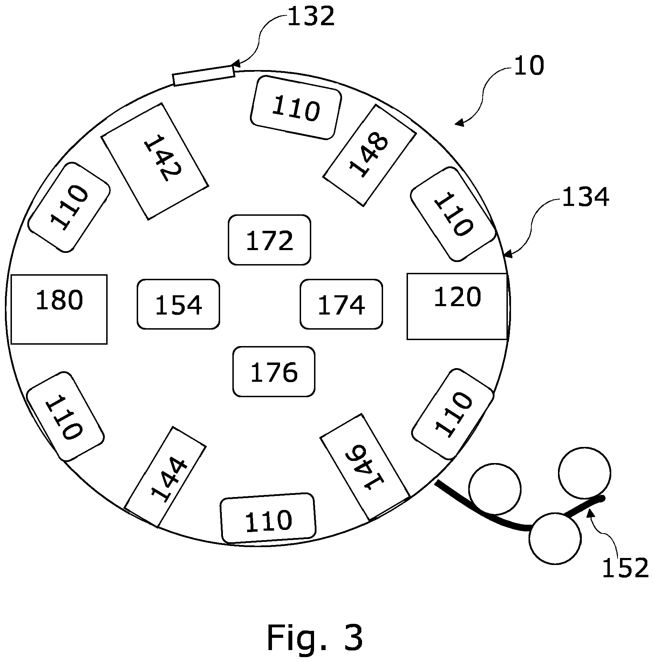

Figure 3 depicts an internal view of an exemplary embodiment of the illumination element 10 according to the present invention. The illumination element 10 may comprise a light source 110. Preferably, the illumination element 10 may comprise a plurality of light sources 110. The light source 110 may comprise, for example, any of a light-emitting diode (LED), a quantum, a laser, or any other suitable light source. The light source 110 may be configured to emit, at least significantly, monochromatic radiation. In other words, the light source 110 may be configured to be a narrow-band light source. The monochromaticity of the light source 110 may be of advantage in optimizing the growth of algae as different wavelengths of light may be of benefit at different stages of growth of the algae. For example, light of wavelength in the range 380 - 800 nm may be typical photosynthetically active radiation, whereas light of wavelength in the range 200 - 380 nm may be of advantage in light stress induction or sanitization. Further, light with wavelength in the range 800 - 1000 nm may be of advantage in boosting growth or stress. Thus, generally, it may be of advantage to control a wavelength of light emitted by the light source 110.

The plurality of light sources 110 may each emit light at the same wavelength. This may be of advantage as different illumination elements 10 may be determined to emit light of different wavelengths, each illumination element 10 emitting light, at least significantly, at only one wavelength. Alternatively, the plurality of light sources 110 may emit at different wavelengths. For example, at least some of the plurality of light sources 110 may emit light at a first wavelength (that may be in the range 380 - 800 nm, for example), while at least some other of the plurality of light sources 110 may emit light at a second wavelength (that may be in the range 200 - 380 nm, for example), and yet some other of the plurality of light sources 110 may emit light at a third wavelength (that may be in the range 800 - 1000 nm, for example). However, generally, it may be understood that the plurality of light sources 110 may be appropriately configured (or chosen) to emit light at any number of different wavelengths.

The illumination element 10 may further comprise a control unit 172. The control unit 172 may be configured to control any of the light sources 110. Controlling a light source 110 may be understood to comprise controlling a state (on/off) of the light source 110, a wavelength of light emitted by the light source 110, an intensity of light emitted by the light source 110 (by controlling a current driven through the light source 110, for example), or a flashing frequency of the light source 110. Flashing frequency may be understood to comprise a frequency at which the state of the light source 110 is switched. Flashing light may be of particular advantage for promoting growth of algae. In particular, flashing frequencies in the range defined by 1 and 200 Hz, preferably by 1 and 150 Hz, further preferably by 1 and 100 Hz, may be advantageous.

Any of the light sources 110 may be configured to receive energy from a battery 120 of the illumination element. The battery 120 may comprise a rechargeable battery that may be recharged wirelessly (via electromagnetic induction, for example) or via a cable. The battery 120 may be configured to be charged by the supply station 30. For example, as depicted in Figure 2, illumination elements 10 may be stationed on the supply station 30 and the batteries 120 thereof may be charged wirelessly. Alternatively, or additionally, the illumination element 10 may comprise a plug that may be complementary to a socket of the supply station 30, such that the illumination element 10 may be plugged into the supply station 30. A plug connection may allow faster rates of energy transfer to the battery 120, whereas a wireless connection may allow for greater simplicity and ease-of-use as a plug connection may have to be made water-resistant.

A capacity of the battery 120 may be determined based, at least in part, on an illuminated (or illuminable) surface area of the illumination element 10. For example, if the surface area of illumination is larger, a larger number of light sources 110 may be needed, thus needing a larger battery capacity. In embodiments of the present technology, the battery capacity may be, for example, in the range defined by 0.005 and 50 kWh, preferably by 0.1 and 25 kWh, further preferably by 1 and 10 kWh per m² of illuminated (illuminable) surface area of the illumination element 10.

The illuminated (or illuminable) surface area may be determined, for example, to be equal to the surface area of the illumination element 10. Alternatively, or additionally, the illuminated (or illuminable) surface area may be determined based, at least in part, on a position of the light source 110 (or of each of the plurality of light sources 110) within the illumination element 10 and/or on a level of brightness observed at a defined distance and/or the defined distance from the illumination element 10, or at least a part thereof.

As described above, it may be advantageous to maintain a density of the illumination element 10, at least significantly, identical to that of the reaction medium 410. In particular, the density of the illumination element 10 may be varied over time to, at least significantly, match a density of the reaction medium 410 around the illumination element 10 when the illumination element 10 is immersed therein. The illumination element 10 may, advantageously, comprise a density control unit 142 that may allow controlling a density of the illumination element 10.

The density control unit 142 may be realized in a plurality of ways. For example, the density control unit 142 may comprise a pump and a storage tank, with the pump fluidly connected to the storage tank. The pump may also be configured to be fluidly connected to an environment of the illumination element 10. The density of the illumination element 10 may then be varied by pumping fluid, particularly liquid (such as the reaction medium 410), in or out of the storage tank. By precisely controlling a volume of the fluid pumped, a precise control over the variation in density may be achieved.

Alternatively, or additionally, the density control unit 142 may comprise a movable cylinder and/or a stretchable element and/or a membrane that may allow changing a specific density of the illumination element 10. Generally, it may be understood that any well-known method for controlling the density may be employed. In particular, any of the methods described above, or a combination of those methods, may be employed.

The density may be controlled by the control unit 172. The control unit 172 may be appropriately configured therefor. For example, the control unit 172 may be configured to at least send data related, at least in part, to a target density to the density control unit 142. For example, the control unit 172 may send data related to a volume of liquid to be pumped out to the density control unit 142.

The illumination element 10 may further comprise a sensor 180. The sensor 180 may comprise, for example, a density sensor that may be configured to determine a density of the reaction medium 410 in an immediate vicinity of the illumination element 10. The sensor 180 may be configured to send data to a data processing unit 174 of the illumination element 10. For example, the density sensor may send the data related to the determined density of the reaction medium 410 in an immediate vicinity of the illumination element 10.

The data processing unit 174 may be configured to communicate (i.e., exchange data) with the control unit 172. For example, the data processing unit 174 may send the data related, at least in part, to the density of the reaction medium 410 in the immediate vicinity of the illumination element 10, to the control unit 172. The data sent may comprise, for example, data related to a volume of liquid to be pumped to, at least significantly, match the density of the reaction medium 410.

An advantageous aspect of the illumination element 10 according to the present invention may be the ability to install different sensors 180 within the illumination element 10. These sensors 180 may allow monitoring of an environment around the illumination element 10, thus allowing monitoring the growth, for example, of algae. Further, the sensors 180 may allow monitoring conditions within the illumination element 10 allowing control, for example, of light emitted by the light source 110. For example, the sensor 180 may comprise a temperature sensor, preferably a plurality of temperature sensors, configured to measure a temperature of the reaction medium 410 in the immediate vicinity of the illumination element 10, as well as a temperature inside the illumination element 10. The measured temperatures may be sent to the data processing unit 174.

The data processing unit 174 may be configured to store (for example, in a memory unit also comprised in the illumination element 10), or at least access (for example, from a remote device), a threshold associated with the sensor 180. The data processing unit 174 may be further configured to carry out a defined action when the data received from the sensor 180 crosses the threshold associated with the sensor 180. For example, the defined action may comprise generating a notification.

The data processing unit 174 may be configured, for example, to send data to the control unit 172 related, at least in part, to switching off of the light source 110 in response to the temperature within the illumination element 10, measured by the temperature sensor 180, exceeding a certain threshold. The certain threshold may be chosen, for example, so as to reduce impact on growth of algae within the container 40. Thus, the data processing unit 174 may allow, at least in part, autonomous control of the illumination element 10.

The illumination element 10 may further comprise a heat exchanger 144. The heat exchanger 144 may be of advantage in regulating a temperature inside the illumination element 10, for example. The heat exchanger 144 may comprise a passive and/or an active heat exchanger. In some embodiments, the illumination element 10 may comprise both a passive and an active heat exchanger. Thus, it may be understood, that the heat exchanger 144 may comprise a plurality of heat exchangers 144. An active heat exchanger 144 may be of particular advantage as a state (on/off) of the heat exchanger may be controlled by the control unit 172. For example, the heat exchanger 144 may be activated when the temperature inside the illumination element 10 exceeds a certain threshold. The control of the active heat exchanger 144 may be similarly mediated by the data processing unit 174, as described above.

The sensor 180 may comprise, for example, a battery capacity sensor that may be configured to determine a remaining battery capacity in the battery 120. Any suitable sensors known in the art may be used therefor. The battery capacity sensor 180 may also send data to the data processing unit 174, wherein the data processing unit 174 may be configured, for example, to generate a notification when the remaining battery capacity falls below a defined threshold (the threshold associated with the battery capacity sensor 180 in this case). The illumination element 10 may comprise a communication unit 176 that may be configured to communicate with the data processing unit 174.

The communication unit 176 may receive, for example, data from the data processing unit 174 related, at least in part, to a crossing of the threshold by data received from a sensor 180. Alternatively, or additionally, the communication unit 176 may receive the data received by the data processing unit 174 from the sensor 180 without additional processing and the communication unit 176 may be configured to send the data as is.

The communication unit 176 may further be configured to communicate with an external device/element. The external device may comprise, for example, the supply station 30, or a remote data processing system such as a computer. The external device may also comprise another illumination element 10.

The communication unit 176 may be configured to communicate by means of electromagnetic radiation with a wavelength suitable for transmission through the reaction medium 410 such as ultraviolet radiation. Alternatively, or additionally, the communication unit 176 may be configured to communicate by means of acoustic radiation, i.e., by means of pressure modulation. For example, the communication unit 176 may comprise a piezoelectric element that may be configured to generate mechanical vibrations in response to an electrical impulse.

In particular, the communication unit 176 may be configured, for example, to receive data related, at least in part, to a low remaining battery capacity of the battery 120, and may be configured to send data related, at least in part, to the low remaining battery capacity to the external device. Thus, advantageously, a state of activity of the illumination element 10 may be monitored even remotely, for example.

The sensor 180 may further comprise any of a position sensor (for determining a position of the illumination element 10 relative to the container 40 and/or to another illumination element 10), a pH sensor, a pressure sensor, a COz and/or O₂ concentration sensor, a brightness sensor, a viscosity sensor, a turbidity sensor, a density sensor, a conductivity sensor, an impedance sensor, an optical density sensor, a photosynthetically active radiation (PAR) sensor, a camera, a microscope, an IR/Raman-spectroscopy-sensor, a cell potential sensor, a potentiostat sensor, a cell cytometry sensor, a specific ion-sensor (e. g., Na⁺, Cl⁻), a specific nutrient concentrations sensor, a photometer sensor, a proximity sensor (to detect a wall of the container), and a proximity sensor/distance sensor between two illumination elements 10. As may be appreciated, different sensors 180 may allow extracting different information about the illumination element 10 and/or its environment, and the information may be used to optimize conditions for growth of the algae.

Figure 3 further depicts an additive dispensing unit 146. The additive dispensing unit 146 may be configured to dispense an additive into the reaction medium 410. The additive may comprise any of CO₂, H₂O₂, O₃, or any other desired additive. Adding additives may allow further control over growth of algae. For example, ozone (Os) and hydrogen peroxide (H₂O₂) may be used for sanitization or oxidative stress induction purposes.

The illumination element 10 may further comprise a sampling unit 148. The sampling unit 148 may be of advantage in retrieving a sample from the reaction medium 410 for analysis. For example, the sampling unit 148 may comprise a storage tank, that may be coupled to a pump. A sample may be drawn into the storage tank. The sampling unit 148 may further be configured to release a sample stored, for example, in the storage tank, into the supply station 30. The sample may be analyzed later on to determine, for example, a composition of the sample, that may allow determining the progress of a growth curve of the algae within the container 40.

Figure 3 further depicts the illumination element 10 comprising a fluorescent tag 152. The fluorescent tag 152 may comprise a fluorescent paint, that may allow the illumination element 10 to fluoresce. The fluorescent tag may be of advantage in detecting the illumination element 10 by external stimulation. For example, the fluorescent tag 152 may be of particular advantage in detecting the illumination element 10 when the battery 120 has run out, so that there is no light emitted from the illumination element 10.

The illumination element 10 may further comprise an identification tag 154. The identification tag may comprise any of an RFID tag, an NFC tag, or any other suitable tag. The identification tag 154 may allow for automatic identification of the illumination element 10, for example, via radio-wave transmission.

As may be appreciated by the skilled person, at least some of the different components of the illumination element 10, as described above, may be powered, at least in part, by the battery 120.

The illumination element 10 may be further configured to be attracted by the supply station 30. The attraction may be of advantage in enabling, at least in part, autonomous control of the illumination elements 10. The illumination element 10 may comprise an attachment element 132 configured to allow the illumination element 10 to be attracted to the supply station 30. For example, the attachment element 132 may comprise a magnet, and the supply station 30 may comprise a complementary magnet. The magnet of the attachment element 132 may comprise a permanent magnet and/or an electromagnet. The electromagnet may be powered by the battery 120, for example. A permanent magnet may be of advantage in reducing energy consumption. An electromagnet may, however, allow better control over the illumination element 10 in conjunction with the supply station 30, for example, as described later.

Figure 3 further depicts a housing 134 of the illumination element 10. The housing 134 may comprise any of a metal (e.g., stainless steel 1.4301, 1.4404, 1.4462, 1.4571), a plastic (e.g., PVC, PMMA, Acryl), silicone, glass (e.g., borosilicate glass, quartz), polymer, ceramics, composite, biomaterial (e.g., wood), a semiconductor, a glass-lined material (e.g., according to ISO 28721-1), or a coated material, as described above. Further, the housing 134 may comprise a material that may be rigid or flexible (inflatable/expandable/stretchable). In particular, the housing 134 may comprise a material that is, at least partially, inert and/or impermeable to the reaction medium 410.

The housing 134 may comprise a plurality of sections and the plurality of sections may be releasably attached to each other. This may allow access to the interior of the illumination element 10. The releasable attachment may be provided, for example, by means of a thread connection, or a bayonet connection. The plurality of sections may comprise, for example, 2 sections. If the illumination element 10 comprises a spherical shape, the 2 sections may each comprise a hemispherical shape that may be attached to each other by means of threads provided on an exterior surface of one of the 2 sections and complementary threads provided on an interior surface of the other of the 2 sections.

At least some of the components of the illumination element 10 described above may be disposed within the housing 134. However, the light source 110 may be disposed within the housing 134 (as depicted in Figure 3), or may be embedded within the housing 134, or may even be arranged on an exterior of the housing 134. For example, the light source 110 may comprise a bead of LEDs that may be wrapped around the housing 134. The light source 110 may, in this case, be understood to be, at least partially, inert and/or impermeable to the reaction medium 410. When the light source 110 is disposed within the housing 134, or is embedded in the housing 134, a material of the housing 134 may be, at least partially, transparent to light emitted by the light source 110. Further, when the sensor 180 comprises a sensor configured to detect a radiative signal (i.e., a signal received via radiation such as electromagnetic radiation), the material of the housing 134 may be, at least partially, transparent to such radiative signals.

Thus, overall, embodiments of the present technology may provide an illumination element 10 that may allow converting any container 40 into a photobioreactor and that may be simpler, easier to install, and may allow greater monitoring and control over the cultivation process.

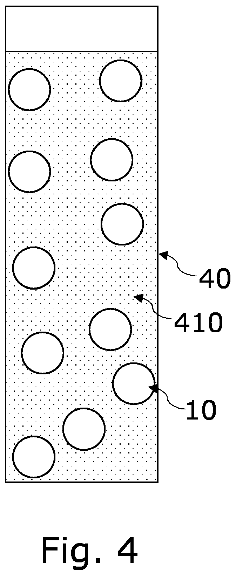

Figure 4 depicts yet another embodiment of a container 40 comprising a reaction medium 410 and a plurality of illumination elements 10. As depicted in Figure 4, owing to the density control unit 142, the density of the illumination elements 10 may be controlled precisely to allow their placement at any height in the reaction medium 410.

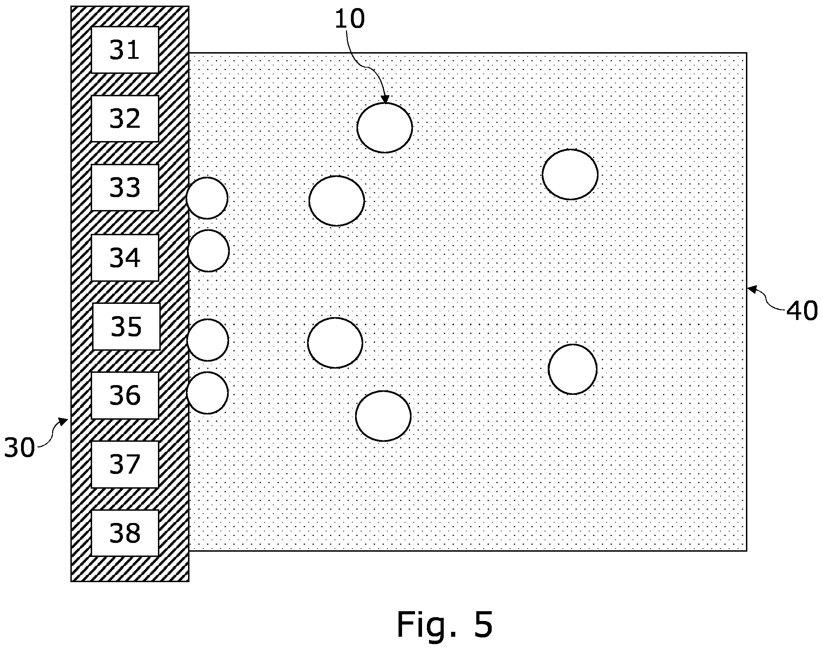

The horizontal positioning of the illumination element 10 within the reaction medium 410 may be achieved, for example, by means of the supply station 30, as depicted in Figure 5. As described above, the illumination element 10 may comprise an attachment element 132 that may allow the illumination element to be attracted by the supply station 30. As also described above, the attachment element 132 may comprise a magnet 132. The supply station 30 may comprise a complementary magnet 320. The supply station magnet 320 may preferably comprise an electromagnet. However, in some embodiments, the supply station magnet 320 may comprise a permanent magnet.

The supply station 30 may further comprise a magnet control unit 310. The magnet control unit 310 may be configured to control, i.e., activate/deactivate, the supply station magnet 320. By controlling the state of the electromagnet 320, the supply station 30 may allow horizontal movement of the illumination element 10 within the reaction medium 410. Further details of the horizontal positioning are described later.

The supply station 30 may comprise a supply station communication unit 330. The supply station communication unit 330 may be configured to communicate with the communication unit 176 of any of the plurality of illumination elements 10. The communication between the supply station 30 and the illumination element 10 may be of particular advantage in allowing horizontal positioning of the illumination element 10. For example, in order to position a defined illumination element 10 horizontally, the supply station communication unit 330 may send a request to the communication unit 176 of the defined illumination element 10 to activate the electromagnet 132 thereof (while, for example, at the same time sending a request to all other illumination elements 10 to deactivate their respective electromagnets 132). The supply station electromagnet 320 may be activated, at least significantly, at the same time. By controlling a strength and/or a spatial profile of the electromagnetic field generated by the supply station electromagnet 320, for example, a desired position of the illumination element 10 may be achieved.

The supply station 30 may further comprise a supply station data processing unit 340. The supply station data processing unit 340 may, in particular, be configured to communicate with the supply station communication unit 330. The supply station data processing unit 340 may be of particular advantage in coordinating the horizontal positioning process as described above, for example. The supply station data processing unit 340 may further be configured to receive and process data from any of the plurality of illumination elements 10. The data may be received via the supply station communication unit 330, for example.

The data received and processed by the supply station data processing unit 340 may comprise, for example, sensor data, or at least data related thereto, measured by the sensor 180 of any of the plurality of illumination elements 10. For example, the data received may comprise data related to a low remaining battery capacity of an illumination element 10, as described above. The data may further comprise data related, at least in part, to a position and/or identification of the illumination element 10. The supply station data processing unit 340 may be configured, in response to receiving the data related to a low remaining battery capacity, effect attraction and attachment of the illumination element 10 to the supply station 30, by means of the electromagnet 320 as described above. Further, the supply station 30 may be configured to release an illumination element 10 with a charged battery 120 to replace the illumination 10 with the low remaining battery capacity.

Various other analyses may be carried out by the supply station data processing unit 340. In particular, the supply station communication unit 310 may be configured to communicate with an external device, such as a remote computer. The data received from any of the illumination elements 10, or at least data related thereto, may be sent to the external device for further analysis. The external device may comprise a data processing capacity that may, for example, be significantly larger than that of the supply station data processing unit 340 or of the illumination element data processing unit 172, thus allowing more complex analyses to be performed. The analyses of data received from the illumination element 10 may be of advantage in optimizing the growth process of the algae.

The supply station 30 may be of particular advantage for charging the battery 120 of any of the plurality of illumination elements 10. Energy may be supplied to the battery 120 wirelessly, by means of electromagnetic induction, or by means of a cable. The supply station may comprise, for example, a supply station induction coil that may be used for wireless charging of the illumination element battery 120. Or, for example, the supply station 30 may comprise a socket complementary to a plug of the illumination element 10 for energy supply over a cable. Preferably, the supply station 30 may be configured to allow simultaneous charging of a plurality of illumination elements 10. For example, the supply station 30 may comprise a plurality of sockets and/or a plurality of supply station induction coils. As may be appreciated by the skilled person, in some embodiments, both methods of energy supply may be provided.

The supply station may further comprise an input terminal 350 for drawing energy from a local grid, for example. A local grid may be understood to comprise energy that may be distributed over power lines, for example, but may also comprise energy generated via renewable sources such as from photovoltaic cells deployed in a local/same building, or from wind turbines, etc.

The energy drawn from the local grid may be supplied to charge the illumination element 10 directly. Alternatively, or additionally, energy drawn from the local grid may be used to charge a supply station battery 360, that may then be used to charge the illumination element 10. The use of a supply station battery 360 may be of particular advantage, for example, if the container 40 is located in a region where supply from a local grid is not, at least continuously, available (as in the case of renewable sources or remote locations, for example). The supply station battery 360 may, for example, be easily replaceable, allowing the supply station 30 and the illumination elements 10 to be used for realizing photobioreactors widely. In some embodiments, for example, the input terminal 350 may not be realized, and the supply station 30 may only supply energy using a replaceable supply station battery 360.

The supply station 30 may further comprise a sample analysis unit 370. The sample analysis unit 370 may be configured to retrieve a sample from the illumination element 10 for analysis, as described above. For example, the sample analysis unit 370 may comprise a sample reservoir that may be coupled to a pump.

The supply station 30 may further comprise an additive dispensing unit 380. The additive dispensing unit 380 may be configured to supply an additive to the illumination element 10. The additive dispensing unit 380 may comprise, for example, a reservoir of an additive. The reservoir may be coupled to a pump.

In particular, any of the components of the supply station 30 may communicate with the supply station data processing unit 340 that may allow for realization of different functionalities of the supply station 30. For example, the sample analysis unit 370 may communicate the retrieval of a sample from an illumination element 10 to the supply station data processing unit 340. The supply station data processing unit 340 may communicate, in response thereto, the retrieval of the sample to the external device. Similarly, the additive dispensing unit 380 may communicate the emptying of an additive reservoir to the supply station data processing unit 340 that may, in response, forward it to the external device. Yet further exemplarily, the supply station 30 may be configured for automatic monitoring of any of the additive/sample reservoir and the supply station data processing unit 340 may be configured for a defined action whenever a level of contents in the reservoir crosses a pre-defined threshold.

Thus, as described above, the supply station 30 according to the present technology may allow for charging of the illumination element 10 according to the present technology, and may also provide for various analyses that may be of advantage in optimizing the growth of algae in the container 40.

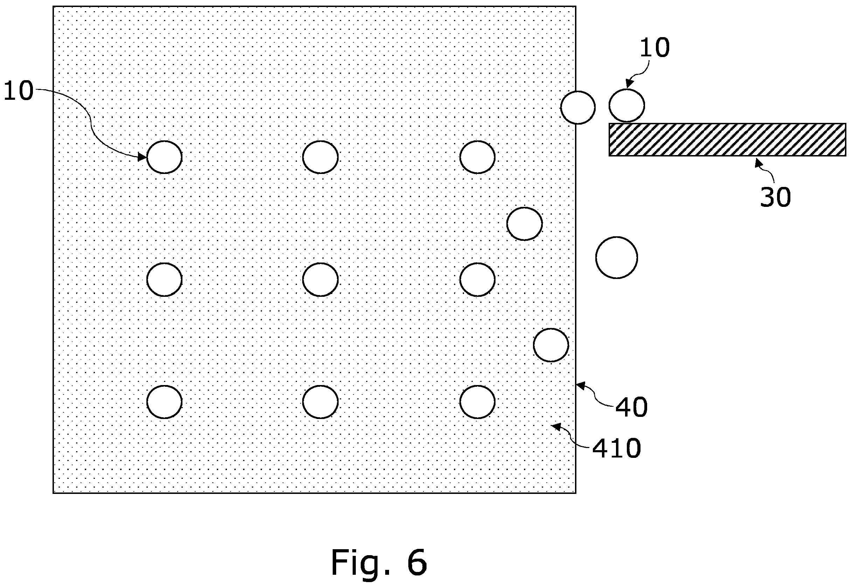

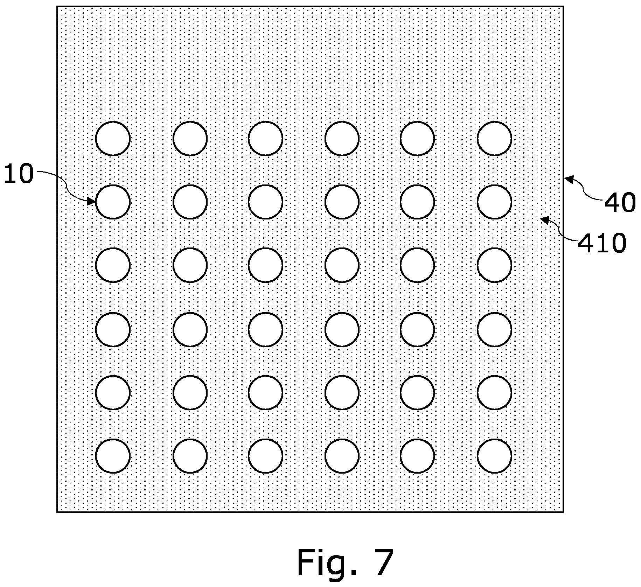

Figures 6 and 7 depict two embodiments of the container 40 comprising a plurality of illumination elements 10 and with two levels of growth of algae, depicted as a varying density of the reaction medium 410. Figure 6 depicts a situation with a low level of growth of algae that may correspond, for example, to an initial stage of cultivation. Figure 7 depicts a high level of growth of algae that may correspond, for example, to a later stage of cultivation. In the initial stage of cultivation, a lower number density of illumination elements 10 may be needed to provide sufficient illumination for growth of algae, as depicted in Figure 6.

For example, Figure 6 may depict a container 40 with dimensions of 1.5 x 1.5 x 1.5 m³, with a filling height of ~ 1.2 m. The container 40 may be equipped with a medium for algae growth with a gas supply (COz) and an agitator (such as a pump, as described above). The container 40 and/or the illumination elements 10 distributed therein may, for example, be further configured for measurement of temperature, O₂ concentration, pH level, and optical density, as described above. As algae grows in the container 40, the above parameters are monitored and depending on the level of growth (determined based, at least in part, on a progress of a growth curve and the monitored parameters, as described above), more illumination elements 10 may be added to the container 40.

Figure 7 depicts the same container 40 at a later time with a higher density of algae in the reaction medium 410. As depicted in Figure 7, the higher density may lead to a higher number density of illumination elements 10 required for optimum illumination (for optimal growth). Thus, a number density of illumination elements 10 may be increased simply by adding in more illumination elements 10. Thus, embodiments of the present technology may allow for optimal cultivation conditions to be realized simply and easily.

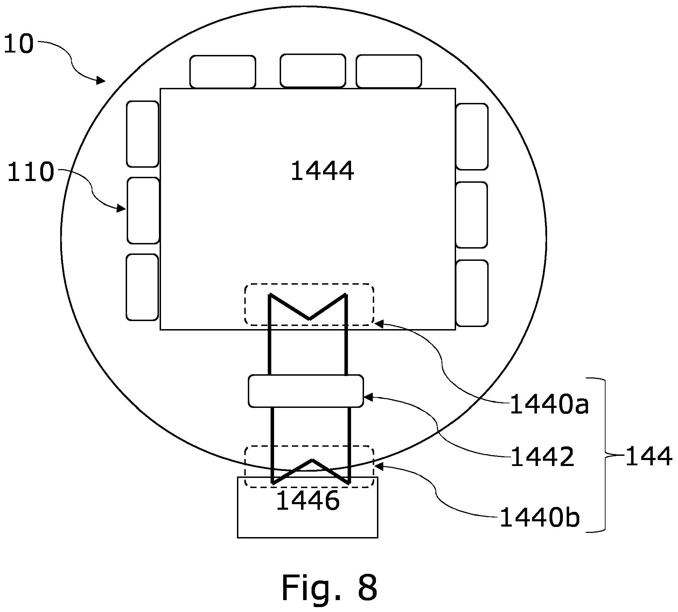

Figure 8 depicts another embodiment of the illumination element 10 comprising an active heat exchanger 144. The depicted embodiment particularly depicts a configuration of a plurality of heat exchanging surfaces 1440a, 1440b in the illumination element 10 (note that the particular embodiment of the illumination element 10 depicted in Figure 8 may also comprise the other components as depicted in the other figures but that are not depicted here). As depicted in Figure 8, the heat exchanging surfaces 1440a, 1440b may be connected to a pump 1442. The pump 1442 may allow pumping, for example, a working fluid through the heat exchanging surfaces 1440a, 1440b. The pumping of the working fluid may thus allow heat flow between a heat source 1444 and a heat sink 1446. The pump 1442 may be controlled by the control unit 172 of the illumination element 10, for example. The heat source 1444 may comprise, for example, a frame and/or a surface on which the light source(s) 110 may be arranged. The frame and/or the surface may comprise a material that has a high thermal conductivity such as aluminum or, generally, any other metal. A further advantage of using aluminum may be a low density allowing for a lower overall weight of the illumination element 10. The thermal conductivity may, for example, be at least 100 W/m/K, preferably at least 150 W/m/K, further preferably at least 200 W/m/K. The heat sink 1446 may, for example, be configured to dissipate heat into the reaction medium 410. Alternatively, or additionally, the heat sink 1446 may comprise a heat storage material that has a high thermal capacity such as lithium nitrate, sodium acetate, sodium acetate trihydrate, or a phase change material (that may, for example, change phase by absorbing heat) that may be configured to store heat received from the heat source 1444. The thermal capacity may, for example, be at least 1 J/(g*K), preferably at least 2 J/(g*K), further preferably at least 3 J/(g*K). The heat storage material may be cooled and/or replaced by retrieving the illumination element 10 from the reaction medium 410, for example.

Overall, embodiments of the present technology thus provide elements, systems, and methods, that may allow conversion of any container into a photobioreactor more simply, easily, and efficiently. Further, embodiments of the present technology may allow more robust, simple, and efficient, monitoring and control of the growth process of biological species in the container.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

While in the above, preferred embodiments have been described with reference to the accompanying drawings, the skilled person will understand that these embodiments were provided for illustrative purpose only and should by no means be construed to limit the scope of the present invention, which is defined by the claims.

## Claims

1. Use of an illumination element (10) in a photobioreactor, wherein the illumination element is adapted for illuminating an interior of a container (40), wherein the illumination element comprises:
a light source (110), and
a battery (120) configured to supply energy to the light source, wherein the battery is a rechargeable battery.

2. Use according to the preceding claim, wherein the illumination element is configured to be connected to a supply station (30), wherein the battery is configured to receive energy from the supply station.

3. Use according to the preceding claim, wherein the energy is received wirelessly.

4. Use according to any of the preceding claims, wherein the illumination element comprises a data processing unit (174).

5. Use according to any of the preceding claims, wherein the illumination element comprises a control unit (172), wherein the control unit is configured to control the light source.

6. Use according to the preceding claim, wherein the control unit is configured to receive data from the data processing unit, and wherein the control unit is configured to control the light source based, at least in part, on the data received from the data processing unit.

7. Use according to any of the preceding claims and with the features of claim 4, wherein the illumination element further comprises a communication unit (176) configured to send data to and/or receive data from an external device/element, and wherein the communication unit (176) is configured to send data to and/or receive data from the data processing unit.

8. Use according to any of the preceding claims, wherein the illumination element further comprises a sensor (180).

9. Use according to the preceding claim and with the features of claim 4, wherein the sensor is configured to send data to the data processing unit.

10. Use according to any of the preceding claims, wherein the container comprises a reaction medium, and wherein the illumination element is configured to be, at least partially, submerged in the reaction medium, and wherein a density of the illumination element is, at least significantly, similar to a density of the reaction medium, or at least a part thereof.

11. Use according to any of the preceding use claims, wherein the illumination element comprises a heat exchanger.

12. A supply station for supplying energy to an illumination element, wherein the supply station is configured to be connected to the illumination element, and wherein the illumination element comprises
a light source, and
a battery configured to supply energy to the light source, wherein the battery is a rechargeable battery.

13. Use of a system in a photobioreactor, wherein the system is adapted for illuminating an interior of a container, wherein the system comprises:
a plurality of illumination elements, wherein each of the plurality of illumination elements comprises
a light source, and
a battery configured to supply energy to the light source, wherein the battery is a rechargeable battery.

14. Use according to the preceding claim, wherein the system further comprises a supply station according to claim 12.

15. A method for illuminating an interior of a container, wherein the method comprises providing a plurality of illumination elements, wherein each of the plurality of illumination elements comprises
a light source, and
a battery configured to supply energy to the light source, wherein the battery is a rechargeable battery.
